# EUROPEAN PATENT SPECIFICATION

(11) **EP 4 045 503 B1**
(45) Date of publication and mention of the grant of the patent: **06.12.2023**
(21) Application number: 20792415.0
(22) Date of filing: 16.10.2020
(51) Int. Cl.: C07D 471/04, A61K 31/437, A61P 35/00

(54) **ESTROGEN-RELATED RECEPTOR ALPHA (ERR-ALPHA) MODULATORS**
OESTROGEN-VERWANDTE REZEPTOR-ALPHA (ERR-ALPHA)-MODULATOREN
MODULATEURS DU RECEPTEUR ALPHA ASSOCIE AUX OESTROGENES (ERR-ALPHA)

(30) Priority: 18.10.2019 EP 19204189
(43) Date of publication of application: 24.08.2022
(73) Proprietor: Lead Pharma Holding B.V., 5349 AB Oss (NL)
(72) Inventor: LEMMERS, Jaap Gerardus Henricus, 5349 AB Oss (NL); DERETEY, Eugen, 5349 AB Oss (NL); KLOMP, Johannes Petrus Gerardus, 5349 AB Amsterdam (NL); CALS, Joseph Maria Gerardus, 5349 AB Oss (NL); OUBRIE, Arthur, 5349 AB Oss (NL)
(74) Representative: De Vries & Metman
(86) International application number: PCT/EP2020/079166
(87) International publication number: WO 2021/074365

(56) References cited:
- WO-A1-2008/109727
- DATABASE REGISTRY [Online] CHEMICAL ABSTRACTS SERVICE, COLUMBUS, OHIO, US; 14 April 2011 (2011-04-14), XP002801594, retrieved from STN Database accession no. 1279843-75-2

## Description

### BACKGROUND TO THE INVENTION

Estrogen-related receptor alpha (ERRα) is a 45.5 kilodalton (kDa), 423 amino acid residue protein which belongs to the nuclear receptor (NR) superfamily. This nuclear receptor family comprises 48 genes, coding for DNA-binding transcription factors that are involved in the regulation of diverse function including inter alia homeostasis, reproduction, development and metabolism. The ERR family - the NR3B subgroup - consists of ERRα, ERR-β, and ERR-γ: to date, no endogenous ligands have been identified for any of the ERR isoforms and therefore they are considered orphan receptors.

in Bookout et al. Anatomical profiling of nuclear receptor expression reveals a hierarchical transcriptional network, Cell. 126:789-99 (2006), organism-wide expression profiling of the three ERR isoforms determined that ERRα is widely distributed, with significant protein expression in most adult tissues. Knockout studies of the ERR family members have revealed that each receptor has tissue- and function-specific metabolic phenotypes that are important for adaptation to energy stress at the whole body level. Knockout studies have also indicated limited *in vivo* compensation among the ERR family members. The disclosure of *inter alia* Tremblay et al. The NR3B subgroup: an overview, Nuclear Receptor Signaling, 5:e009 (2007) may be noted in this context.

Genomic studies have indicated that ERRα regulates large numbers of genes. The following references are instructive in this regard: Puigserver et al. A cold-inducible coactivator of nuclear receptors linked to adaptive thermogenesis, Cell. 92(6):829-839 (1998); Yoon et al. Control of hepatic gluconeogenesis through the transcriptional coactivator PGC-1, Nature 413(6852):131-138 (2001); Huss et al. Estrogen-related receptor alpha directs peroxisome proliferator-activated receptor at signaling in the transcriptional control of energy metabolism in cardiac and skeletal muscle, Mol. Cell Biol. 24(20):9079-9091 (2004); and, Mootha et al. ERRα and Gabpa/b specify PGC-1alpha-dependent oxidative phosphorylation gene expression that is altered in diabetic muscle, Proc. Natl. Acad. Sci. USA, 101 (17):6570-6575 (2004).

These references support a physiological model of ERRα function in regulating energy metabolism and, in particular, in the transcriptional regulation of genes required for mitochondrial biogenesis, the tricarboxylic acid cycle, oxidative phosphorylation, fatty acid oxidation and lipid metabolism. In particular, ERRα induces the expression of Nuclear Respiratory Factor 1 (NRF1), GA-binding protein alpha (GABPα), and Peroxisome Proliferator-activated Receptor alpha (PPARα). The nuclear receptor coactivators Peroxisome Proliferator-activated Receptor gamma coactivator 1-alpha (PGC-1α), PGC-1 β and Peroxisome Proliferator-activated Receptor gamma Coactivator-related protein 1 (PPRC-1) are implicated in the regulation of these genes and in the auto-regulation of the expression of ERRα. PGC-1α is expressed at low basal levels but is induced by fasting and other metabolic stresses. PGC-1β, a related coactivator, has similar functions, but its expression may not be regulated as acutely by variations in energy demand. Conversely, co-repressors that bind to ERRs, such as co-repressor nuclear Receptor Interacting Protein 140 (RIP140), compete with ERR co-activators to negatively regulate ERR-dependent gene expression.

The pleiotropic effect of ERRα activity on energy metabolism has interested the present inventors in the possibility that it should be a target for the discovery of new therapies for diseases in which metabolic disturbances or modifications play a central role, such as type-2 diabetes, progressive heart failure, osteoporosis and cancer. Of particular interest is ERRα as a novel target for tumor therapy, through effects on the regulation of tumor cell energy metabolism associated with energy stress within tumor microenvironments. And of specific interest, is ERRα as a novel target for therapeutic treatment of cancers with stem-like properties - Cancer Stem Cells (CSC), Tumor Initiating Cells (TIC) and Circulating Tumor Cells (CTC) - that rely on mitochondrial respiration for their energy requirements.

The initiation and development of cancer, in particular, is known to be associated with major metabolic alterations and mitochondria play a key role in tumorigenesis. A common abnormality observed in many cancer types - termed the Warburg effect - is a shift in glucose metabolism from oxidative phosphorylation to aerobic glycolysis and is characterized by a drastic increase in glucose consumption accompanied by an elevated rate of lactate excretion regardless of oxygen abundance: aerobic glycolysis meets the metabolic needs of highly proliferative cells, including providing sufficient energy and providing for the accumulation of precursors for anabolic reactions. LeBleu et al. PGC-1alpha mediates mitochondrial biogenesis and oxidative phosphorylation in cancer cells to promote metastasis, Nat. Cell Biol. 16(10):992-1 (2014) demonstrated that tumor cells display metabolic plasticity to engage either glycolysis or oxidative phosphorylation depending on the tumor environment and their proliferative or metastasizing phenotype during cancer progression. It is thus evident that the targeting of metastatic progenitors and resistant tumor cells should not only happen via the glycolytic route but also via the mitochondrial oxidative phosphorylation.

ERRα, together with PGC1α/β, controls the regulation of genes encoding enzymes in the tricarboxylic acid (TCA) cycle and oxidative phosphorylation. As discussed in Ariazi et al. Estrogen-related receptor alpha and estrogen-related receptor gamma associate with unfavorable and favorable biomarkers, respectively, in human breast cancer, Cancer Res. 62(22):6510-8 (2002), ERRα is expressed in a range of cancerous cells - including breast and prostate cancerous cells - and is associated with more invasive disease and a higher risk of recurrences in both these cancer types.

Chang et al. The metabolic regulator ERRa, a downstream target of HER2/IGF-1R, as a therapeutic target in breast cancer, Cancer Cell 20, 500-510 (2011) and Fujimoto et al. Clinical implication of estrogen-related receptor (ERR) expression in ovarian cancers, J. Steroid Biochem. Mol. Biol. 104, 301-304 (2007) document that ERRα is expressed in most cancers and that increased activity of this receptor is associated with a negative outcome in both breast and ovarian cancers. In the first of these references, it is confirmed that the transcription factor is involved in mitochondrial biogenesis and also in the regulation of oxidative phosphorylation. This latter point is considered important as resistance to the inhibition of KRAS pathway in pancreatic cancer, BRAF inhibitors in melanoma and oxaliplatin and 5-fluorouracil in colon cancer are also associated with a shift to oxidative metabolism.

The present inventors have therefore opined that inhibition of the activity of ERRα would enable a selective disruption of mitochondrial function in cancer, in particular in cancers of the aforementioned types. For this purpose, but equally for utility in the treatment of other ERRα mediated diseases and conditions, they have developed non-covalent, non-steroidal ERRα inverse agonists.

WO 2008/109727 describes substituted phenoxy thiazolidinediones as ERRα modulators.

### SUMMARY OF THE INVENTION

In accordance with a first aspect of the present invention, there is provided a compound according to Formula I 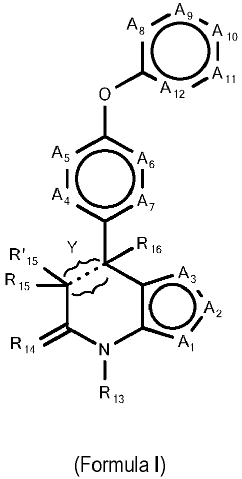 or a pharmaceutically acceptable salt thereof, wherein
Y is a single carbon-carbon bond or a double carbon-carbon bond, with the proviso that when Y is a double carbon-carbon bond, R'₁₅ and R₁₆ are not present;
one of the three positions A₁-A₃ is either S or NR_{A}, the remaining two positions A₁-A₃ are N or CR₁, CR₂, CR₃, respectively;
R_{A} is H or methyl;
R₁-R₃ are independently H, methyl, amino or halogen;
A₄-A₇ are CR₄, CR₅, CR₆ and CR₇, respectively;
A₈-A₁₂ are N or CR₈, CR₉, CR₁₀, CR₁₁ and CR₁₂, respectively, with the proviso that no more than two of the five positions A₈ - A₁₂ can be simultaneously N;
R₄ - R₇ are independently H, halogen, C(1-3)alkoxy, C(1-3)alkyl;
R₈ - R₁₂ are independently H, halogen, C(1-3)alkoxy, C(1-4)alkyl, cyano, nitro, -C(=O)OR₁₇ , hydroxyl, C(3-6)cycloalkyl, benzyl, phenyl, -SF₅, bicyclo[1.1.1]pentane;
or R₉ and either R₈ or R₁₀ are fused and form an aromatic or non-aromatic five to seven membered ring containing two to seven carbon atoms and zero to three hetero atoms; with all groups optionally substituted with one or more methyl, halogen or hydroxyl;
R₁₃ is H or methyl;
R₁₄ is NH, O or S;
R'₁₅ is H, halogen, C(1-4)alkyl, -C(=O)OR₁₇ or-C(=O)NR₁₇R₁₇;
R₁₅ H, halogen, or C(1-4)alkyl;
R₁₆ is H; and,
R₁₇ is H, methyl or ethyl.

In an embodiment, the invention relates to a compound according to Formula I in which A₁ is N, A₂ is NR_{A} and A₃ is CR₃.

In another embodiment, the invention relates to a compound according to Formula I in which A₁ is N, A₂ is NH and A₃ is CH.

In another embodiment, the invention relates to a compound according to Formula I in which R₅ is C(1-3)alkoxy and R₄, R₆ and R₇ are H.

In another embodiment, the invention relates to a compound according to Formula I in which R₅ is methoxy and R₄, R₆ and R₇ are H.

In another embodiment, the invention relates to a compound according to Formula I in which A₈-A₁₂ are CR₈, CR₉, CR₁₀, CR₁₁ and CR₁₂, respectively.

In another embodiment, the invention relates to a compound according to Formula I in which R₈-R₁₂ are independently H, C(1-4)alkyl, halogen, hydroxyl or C(1-3)alkoxy. For instance, R₈-R₁₂ may be independently H, C(1-4)alkyl or halogen. Good results have been obtained where R₉ and R₁₁ are independently C(1-4)alkyl, halogen, hydroxyl or C(1-3)alkoxy and R₈, R₁₀ and R₁₂ are H. And, of particular note, R₉ and R₁₁ are independently C(1-4)alkyl and R₈, R₁₀ and R₁₂ are H.

In another embodiment, the invention relates to a compound according to Formula I in which R₉ and R₁₁ are CF₃ and R₈, R₁₀ and R₁₂ are H.

In another embodiment, the invention relates to a compound according to Formula I in which R₁₃ is H.

In another embodiment, the invention relates to a compound according to Formula I in which R₁₄ is O.

In another embodiment, the invention relates to a compound according to Formula I in which R₁₅ and R'₁₅ are H.

In another embodiment, the invention relates to a compound according to Formula I in which Y is a single carbon-carbon bond.

The above embodiments, where they relate to a preferred form of different substituents of Formula (I), are not intended to be mutually exclusive of one another. Rather, combinations of these embodiments are envisaged within the scope of the present invention and, in certain circumstances, such combinations represent preferred structures for compounds of Formula I. In that regard, particular mention may be made of compounds according to Formula I in which: A₁ is N, A₂ is NH and A₃ is CH; A₄-A₇ are CR₄, CR₅, CR₆ and CR₇ respectively, wherein R₅ is C(1-3)alkoxy and R₄, R₆ and R₇ are H; A₈-A₁₂ are CR₈, CR₉, CR₁₀, CR₁₁ and CR₁₂, respectively, wherein R₉ and R₁₁ are independently C(1-4)alkyl, halogen, hydroxyl or C(1-3)alkoxy and R₈, R₁₀ and R₁₂ are H; R₁₃ is H; R₁₄ is O; R₁₅ and R'₁₅ are both H; Y is a single carbon-carbon bond; and, R₁₆ is H.

Mention may also be made of compounds according to Formula I in which: A₁ is N, A₂ is NH and A₃ is CH; A₄-A₇ are CR₄, CR₅, CR₆ and CR₇ respectively, wherein R₅ is C(1-3)alkoxy and R₄, R₆ and R₇ are H; A₈-A₁₂ are CR₈, CR₉, CR₁₀, CR₁₁ and CR₁₂, respectively, wherein R₉ and R₁₁ are independently C(1-4)alkyl and R₈, R₁₀ and R₁₂ are H; R₁₃ is H; R₁₄ is O; R₁₅ and R'₁₅ are both H; Y is a single carbon-carbon bond; and, R₁₆ is H.

### DEFINITIONS

As used herein, the singular forms "a", *"an"* and "the" include plural referents unless the context clearly dictates otherwise.

The terms *"comprising, "comprises"* and *"comprised of'* as used herein are synonymous with *"including, "includes", "containin"* or *"contains",* and are inclusive or open-ended and do not exclude additional, non-recited members, elements or method steps. If used, the phrase *"consisting of"* is closed and excludes all additional elements. Further, the phrase *"consisting essentially* of excludes additional material elements but allows the inclusion of non-material elements that do not substantially change the nature of the invention.

When amounts, concentrations, dimensions and other parameters are expressed in the form of a range, a preferable range, an upper limit value, a lower limit value or preferable upper and lower limit values, it should be understood that any ranges obtainable by combining any upper limit or preferable value with any lower limit or preferable value are also specifically disclosed, irrespective of whether the obtained ranges are clearly mentioned in the context.

The words *"preferred", "preferably", "desirably"* and *"particularly"* or synonyms thereof may be used frequently herein to refer to embodiments of the disclosure that may afford particular benefits, under certain circumstances. However, the recitation of one or more preferable, preferred, desirable or particular embodiments does not imply that other embodiments are not useful and is not intended to exclude those other embodiments from the scope of the disclosure.

As used throughout this application, the word "may" is used in a permissive sense - that is meaning to have the potential to - rather than in the mandatory sense.

As used herein *"room temperature"* is 23°C ± 2°C.

Unless otherwise stated, the terms "halo" or *"halogen"* or *"halide",* as used herein by themselves or as part of another substituent, mean a fluorine, chlorine, bromine, or iodine atom. A preference for fluorine, chlorine or bromine is noted.

The term *"heteroatom"* as used herein represents nitrogen, oxygen or sulfur.

As used herein, *"C(1-n)alkyl"* group refers to a monovalent group that contains from 1 to n carbon atoms, that is a radical of an alkane and includes straight-chain and branched organic groups. As such, a *"C₁-C₃₀alkyl"* group would refer to a monovalent group that contains from 1 to 30 carbon atoms, that is a radical of an alkane and includes straight-chain and branched organic groups. In the present invention, such alkyl groups may be unsubstituted or may be substituted with the groups mentioned herein below. The halogenated derivatives of hydrocarbon radicals might, in particular, be mentioned as examples of suitable substituted alkyl groups.

The term *"C(1-4)alkyl"* as used herein means an alkyl group having 1-4 carbon atoms, i.e. methyl, ethyl, propyl, isopropyl, butyl, isobutyl, sec-butyl or tert-butyl. All carbon atoms may optionally be substituted with one or more halogen or hydroxyl.

The term *"C(1-3)alkyl"* as used herein means an alkyl group having 1-3 carbon atoms, i.e. methyl, ethyl, propyl or isopropyl. All carbon atoms may optionally be substituted with one or more halogen or hydroxyl.

The term *"C(3-6)cycloalkyP'* as used herein means a saturated cyclic hydrocarbon having 3-6 carbon atoms, i.e. cyclopropyl, cyclobutyl, cyclopentyl or cyclohexyl. All carbon atoms may optionally be substituted with one or more halogen or methyl.

The term *"C(1-3)alkoxy"* means an alkoxy group having 1-3 carbon atoms, the alkyl moiety being branched or unbranched. All carbon atoms are optionally substituted with one or more F or hydroxyl.

The term *"cyano"* as *used herein,* represents a *group* of formula -CN.

As used herein *"nitro group"* or *"nitro"* refers to -NO₂.

The term *"substituted'* means that one or more hydrogens on the designated atom(s) is/are replaced by a selection from the indicated group, provided that: the designated atom's normal valency under the existing circumstances is not exceeded; and, the substitution results in a stable compound. Combinations of substituents are also permissible only if such combinations result in stable compounds. The terms *"stable compound"* or *"stable structure"* refers to a compound or structure that is sufficiently robust to survive both isolation to a useful degree of purity from a reaction mixture and formulation into an efficacious therapeutic agent.

The term *"optionally substituted'* means optional substitution with the specified groups, radicals or moieties.

As used herein *"protecting group"* refers to a moiety attached to a functional group to prevent an undesired reaction. Preferably the protecting group may be easily removed after protection of the functional group is no longer required.

The compounds of Formula I may form salts, which are also within the scope of this invention. Reference to a compound of Formula I herein is understood to include reference to salts thereof, unless otherwise indicated.

The term *"pharmaceutically acceptable salt'* is used in accordance with its standard definition in the art to represent those salts which are, within the scope of medical judgment, suitable for use in contact with the tissues of humans and lower animals without, in particular, undue toxicity, irritation and / or allergic response: that use must be commensurate with a reasonable benefit to risk ratio. Pharmaceutically acceptable salts are well known in the art. They may either be obtained during the final isolation and purification of the compounds of the invention, or they may be obtained separately by reacting a free base function with: a suitable mineral acid, including but not limited to hydrochloric acid, phosphoric acid or sulfuric acid; or, an organic acid, including but not limited to ascorbic acid, citric acid, tartaric acid, lactic acid, maleic acid, malonic acid, fumaric acid, glycolic acid, succinic acid, propionic acid, acetic acid or methanesulfonic acid. An acid function of compounds of the invention can be reacted with an organic or a mineral base, like sodium hydroxide, potassium hydroxide or lithium hydroxide. For completeness, organic bases include the common hydrocarbyl and heterocyclic amine salts, such as diethylamino, morpholine and piperidine salts, for example.

The compounds of Formula I may contain asymmetric or chiral centers and therefore exist in different stereoisomeric forms. It is intended that all stereoisomeric forms of the compounds of Formula I as well as mixtures thereof, including racemic mixtures, form part of the present invention. In particular, stereoisomeric forms of the compounds of Formula I which, following the Cahn-Ingold-Prelog system of nomenclature, are in the S configuration on the chiral center next to the five-membered heterocyclic ring definitively form part of the present invention.

As will be understood by the skilled artisan, enantiomers can be separated by: converting the enantiomeric mixture into a diastereomeric mixture by reaction with an appropriate optically active compound, for instance a chiral auxiliary such as a chiral alcohol or Mosher's acid chloride; separating the diastereomers; and, converting - by hydrolysis for example - the individual diastereomers to the corresponding pure enantiomers. Enantiomers can also be separated by use of chiral HPLC column.

It will be recognized further that various tautomers of compounds of Formula I may be possible: it is therefore intended that all tautomeric forms of compounds of Formula I form part of the invention. For completeness, as used herein, the term "tautomer" refers to the migration of protons between adjacent single and double bonds. The tautomerization process is reversible: tautomers will generally reach an equilibrium state wherein the double bond is resonantly shared between two bond lengths.

The present invention also relates to a pharmaceutical composition comprising compounds or pharmaceutically acceptable salts thereof having the general Formula I in admixture with pharmaceutically acceptable excipients and optionally other therapeutically active agents. The excipients must be "acceptable" in the sense of being compatible with the other ingredients of the composition and not deleterious to the recipients thereof.

The invention further includes a compound of Formula I in combination with one or more other drug(s).

Compositions include, but are not limited to, those suitable for oral, sublingual, subcutaneous, intravenous, intramuscular, nasal, local, or rectal administration, all in unit dosage forms for administration. For oral administration, the active ingredient may be presented as discrete units, such as tablets, capsules, powders, granulates, solutions, suspensions and the like.

For parenteral administration, the pharmaceutical composition of the invention may be presented in unit-dose or multidose containers, such as injection liquids in predetermined amounts, presented for example in sealed vials and ampoules. The pharmaceutical composition may also be stored in a freeze dried (lyophilized) condition requiring only the addition of sterile liquid carrier - such as water - prior to use.

Mixed with such pharmaceutically acceptable auxiliaries, the active agent may be compressed into solid dosage units, such as pills, tablets, or be processed into capsules or suppositories. By means of pharmaceutically acceptable liquids, the active agent can be applied as a fluid composition - in the form of a solution, suspension or emulsion for instance - which may be included in an injection preparation or in a spray, such as a nasal spray.

For making solid dosage units, the use of conventional additives such as fillers, colorants, polymeric binders and the like is contemplated. In general, any pharmaceutically acceptable additive which does not interfere with the function of the active compounds can be used. Suitable carriers with which the active agent of the invention can be administered as solid compositions include lactose, starch, cellulose derivatives and the like, or mixtures thereof, when used in suitable amounts. For parenteral administration, aqueous suspensions, isotonic saline solutions and sterile injectable solutions may be used, which suspensions or solutions may contain pharmaceutically acceptable dispersing agents and/or wetting agents, such as propylene glycol or butylene glycol.

The invention further includes a pharmaceutical composition, as herein before described, in combination with packaging material suitable for said composition, said packaging material including instructions for the use of the composition for the purposes as hereinbefore described.

The exact dose and regimen of administration of the active ingredient, or a pharmaceutical composition thereof, may vary with the particular compound, the route of administration, and the age and condition of the individual subject to whom the medicament is to be administered.

In general parenteral administration requires lower dosages than other methods of administration which are more dependent upon absorption. That aside, a dosage for humans preferably contains from 0.0001 to 100 mg per kg body weight. The desired dose may be presented as one dose or as multiple sub-doses administered at appropriate intervals throughout the day.

The compounds according to the invention or a pharmaceutically acceptable salt thereof can be used as medicament in therapy.

Another aspect of the invention resides in the use of compounds having the general Formula I or a pharmaceutically acceptable salt thereof for the therapeutic and / or prophylactic treatment of ERRa-mediated diseases or ERRα mediated conditions. In particular, the invention provides for the use of compounds having the general Formula I or a pharmaceutically acceptable salt thereof for the treatment of ERRα-mediated cancer.

The compounds having the general Formula I or a pharmaceutically acceptable salt thereof can be used in therapies to treat at least one condition selected from: lung cancer; melanoma; endometrial cancer; and, acute myeloid leukemia. Without intention to limit this aspect of the present invention, the compounds having the general Formula I or a pharmaceutically acceptable salt thereof may in particular be used in therapies to treat: superficial spreading melanoma; lentigo maligna; acral lentiginous melanoma; nodular melanoma; amelanotic melanoma; ocular melanoma; melanoma of the vulva; or, vaginal melanoma.

In another aspect, the compounds having the general Formula I or a pharmaceutically acceptable salt thereof can be used in therapies to treat at least one condition selected from: breast cancer; bladder cancer; prostrate cancer; pancreatic cancer; colorectal cancer; and, ovarian cancer.

In another aspect, the compounds having the general Formula I or a pharmaceutically acceptable salt thereof can be used to treat Type II Diabetes Mellitus.

### DETAILED DESCRIPTION OF THE INVENTION

As depicted in the Examples below, in certain exemplary embodiments compounds are prepared according to the following general procedures. It will be appreciated that, whilst the general methods depict the synthesis of certain compounds of the invention, the following general methods and other methods know to one skilled in the art, can be applied to all compounds and subclasses and species of each of these compounds, as described herein.

### GENERAL METHODS OF PREPARATION

The compounds described herein, including compounds of general Formula I, Building Block I and Building Block II, are prepared by the reaction schemes depicted below. Furthermore, in the following schemes, where specific acids, bases, reagents, coupling agents, catalysts, solvents and the like are mentioned, it is understood that other suitable acid, bases, reagents, coupling agents, catalysts, solvents, etc. may be used and are included within the scope of the present invention. Modifications to the reaction conditions - for example, temperature, duration of the reaction or combinations thereof - are envisioned as part of the present invention.

The compounds obtained by using the general reaction sequences may be of insufficient purity. The compounds can be purified by using any of the methods for purification of organic compounds, for example, crystallization or silica gel or alumina column chromatography using different solvents in suitable ratios. All possible stereoisomers are envisioned within the scope of the invention.

Abbreviations for the materials employed in the Reaction Schemes and Examples are as follows:
**AcOH:** acetic acid; **ACN:** acetonitrile; **DAST:** (Diethylamino)sulfur trifluoride; **DCM:** dichloromethane; **DDQ:** 2,3-Dichloro-5,6-dicyano-1,4-benzoquinone; **DIAD:** Diisopropyl azodicarboxylate; **DiBAI-H:** Diisobutylaluminium hydride; **DMAP:** 4-dimethylaminopyridine; **DMF:** N,N-dimethylformamide; **DMSO:** Dimethyl sulfoxide; **EDC:** 1-Ethyl-3-(3-dimethylaminopropyl)carbodiimide; **Et₂O:** di-ethyl ether; **EtOAc:** ethyl acetate; **EtOH:** Ethanol; **HPLC:** High-performance liquid chromatography **MeOH:** Methanol; **PhSCu(l):** phenylsulfanylcopper; **PPh₃:** Tripenylphosphine; **SFC:** Supercritical fluid chromatography; **tBuOK:** potassium tert-butoxide; **tBuONO:** tert-Butyl nitrite; **TEMPO:** 2,2,6,6-tetramethylpiperidinyloxyl; **TFA:** trifluoroacetic acid; **THF:** tetrahydrofuran; **TOSMIC:** Tosylmethylisocyanide.

Chemical names are preferred IUPAC names, generated by using Marvin Sketch 17.24.1. If a chemical compound is referred to using both a chemical structure and a chemical name, and an ambiguity exists between the structure and the name, the structure predominates.

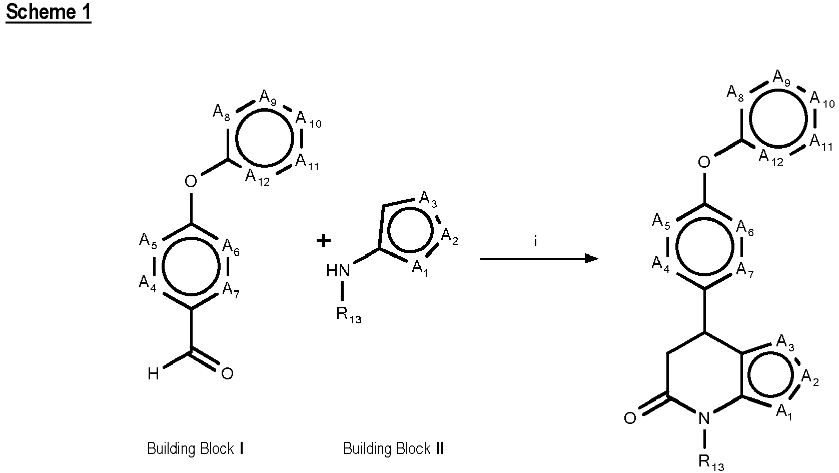

### Conditions: i) Meldrum's acid, MeOH

Depicted in scheme **1** is the general synthesis of derivatives of the invention having Formula I, wherein R₁₄ is oxygen, R₁₅, R'₁₅ and R₁₆ are H and Y is a single carbon-carbon bond. The derivatives can be prepared by methods known in the art of organic chemistry. Compounds of the invention can be obtained by a reaction between a benzaldehyde derivative of building block I, wherein A₄, A₅, A₆, A₇, A₈, A₉, A₁₀, A₁₁ and A₁₂, have the meaning as previously described, and a derivative of building block II, wherein R₁₃, A₁, A₂ and A₃ have the meaning as previously described, and Meldrum's acid.

To obtain derivatives of Formula I wherein R₁₄ is sulphur, the derivatives of Formula I wherein R₁₄ is oxygen can be reacted with, for example, Lawesson's reagent.

To obtain derivatives of Formula I wherein R₁₄ is nitrogen, the derivatives of Formula I wherein R₁₄ is sulphur can be reacted with, for example, ammonia in MeOH.

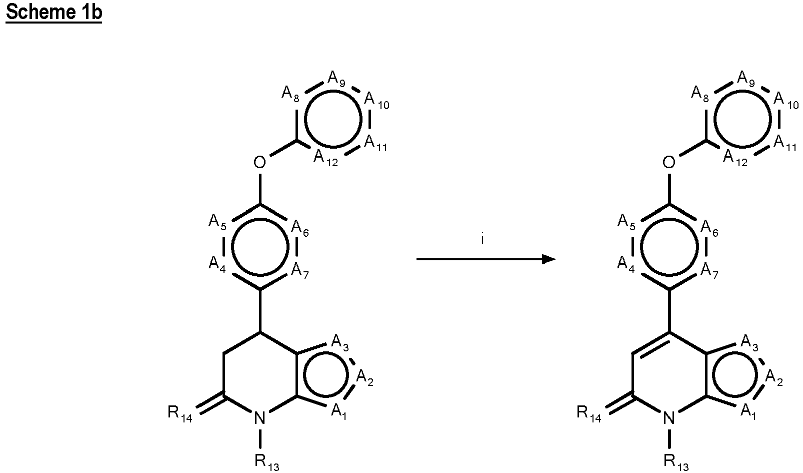

### Conditions: i) DDQ, 1,4-dioxane

Scheme 1b depicts a general route for the preparation of Formula I analogs wherein Y is a double carbon-carbon bond, R₁₅ is H and R₁₃, R₁₄, A₁, A₂, A₃, A₄, A₅, A₆, A₇, A₈, A₉, A₁₀, A₁₁ and A₁₂ have the meaning as previously described.

Derivatives of Formula I, wherein Y is a single carbon-carbon bond and R₁₅, R'₁₅ and R₁₆ are H, can be oxidized, using for example DDQ in an appropriate solvent, to obtain derivatives of Formula I, wherein Y is a double carbon-carbon bond.

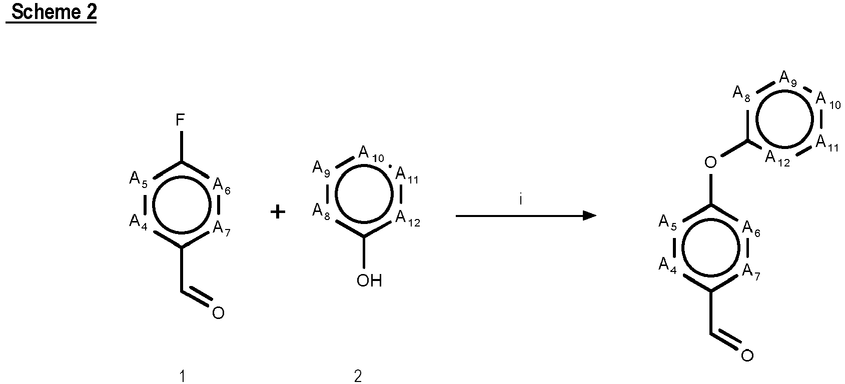

### Conditions: i) K₂CO₃, DMF

Scheme 2 depicts a general method for preparing benzaldehyde building block I derivatives wherein A₄, A₅, A₆, A₇, A₈, A₉, A₁₀, A₁₁ and A₁₂ have the meaning as previously described.

Aromatic substitution of 4-fluorobenzaldehyde **1** with phenol **2,** using K₂CO₃ in DMF at 110 °C, gives the building block I derivatives. For some substitutions on A₄ to A₁₂, it might be beneficial to use a 4-hydroxybenzaldehyde derivative with a fluorobenzene derivative to perform the aromatic substitution.

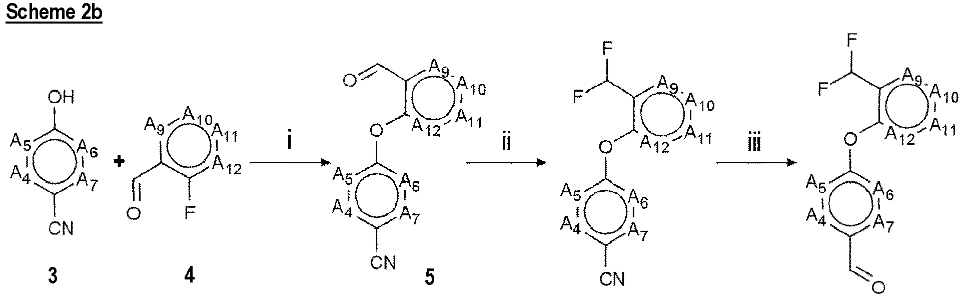

Conditions:
i) K₂CO₃, DMF;
ii) DAST, DCM;
iii) DiBAI-H, toluene.

Scheme **2b** depicts a general method for preparing benzaldehyde building block I derivatives, wherein A₈ is C-CF₂, and A₄, A₅, A₆, A₇, A₉, A₁₀, A₁₁ and A₁₂ have the meaning as previously described. Aromatic substitution of fluorobenzaldehyde **4** with 4-hydroxybenzonitrile **3** under basic condition, using for example K₂CO₃, gives the corresponding benzonitrile **5**. The aldehyde moiety can be converted to a CF₂ group using a fluorinating agent, for example DAST. Via subsequent reduction of the nitrile using, for example DiBAI-H in toluene, benzaldehyde building block I derivatives can be prepared. In scheme **2b** this is exemplified for R₈, these conversion can also be applied for an aldehyde moiety in any of the positions R₄ to R₁₂. For some substitutions on A₄ to A₁₂, it might be beneficial to use a 4-fluorobenzonitrile derivative with a hydroxybenzaldehyde derivative to perform the aromatic substitution.

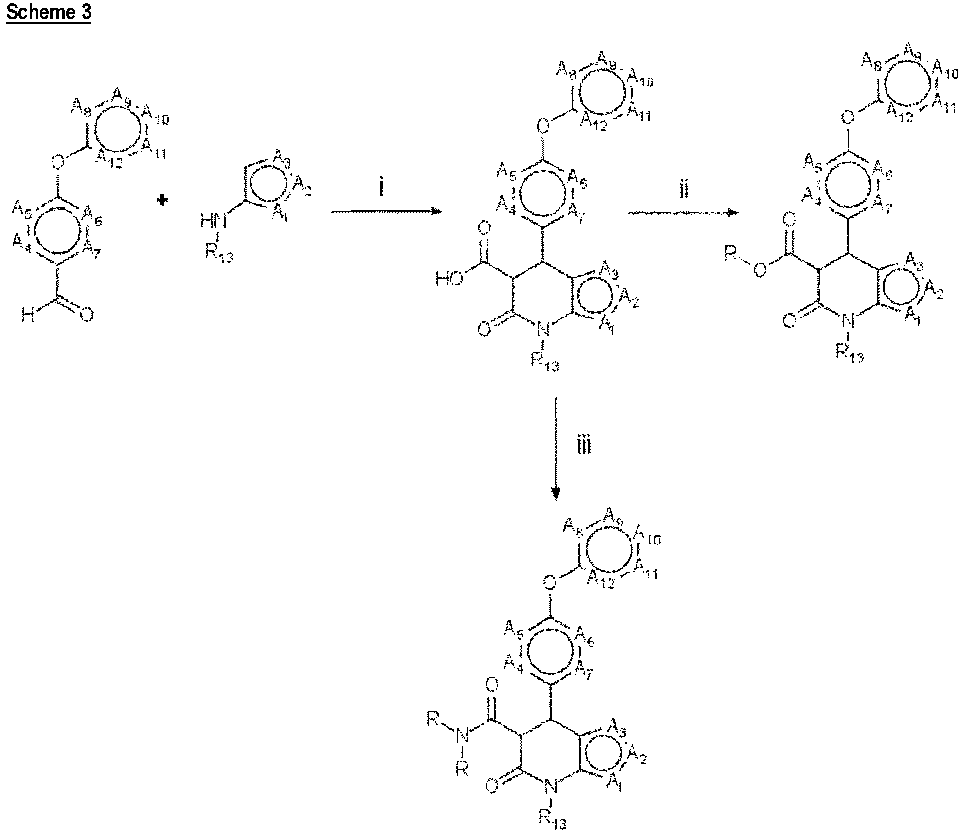

Conditions:
i) Meldrum's acid, MeOH;
ii) ROH, DIAD, PPh₃, THF; and,
iii) R₂NH, EDC, DMAP, DCM.

Scheme **3** depicts a general route for the preparation of Formula **I** analogs wherein Y is a single carbon-carbon bond, R₁₄ is oxygen, R₁₅ is COOH and each of R'₁₅ and R₁₆ is H. Compounds of the invention can be obtained by a reaction between a derivative of building block I, wherein A₄, A₅, A₆, A₇, A₈, A₉, A₁₀, A₁₁ and A₁₂ have the meaning as previously described, a derivative of building block II, wherein R₁₃, A₁, A₂, A₃ have the meaning as previously described, and Meldrum's acid at room temperature.

When R₁₅ is COOH, this carboxylic acid moiety can be functionalized towards an ester, using for example an alcohol, DIAD and PPh₃ in THF. Or it can be functionalized towards an amide, using for example a primary or secondary amine, EDC and DMAP in DCM.

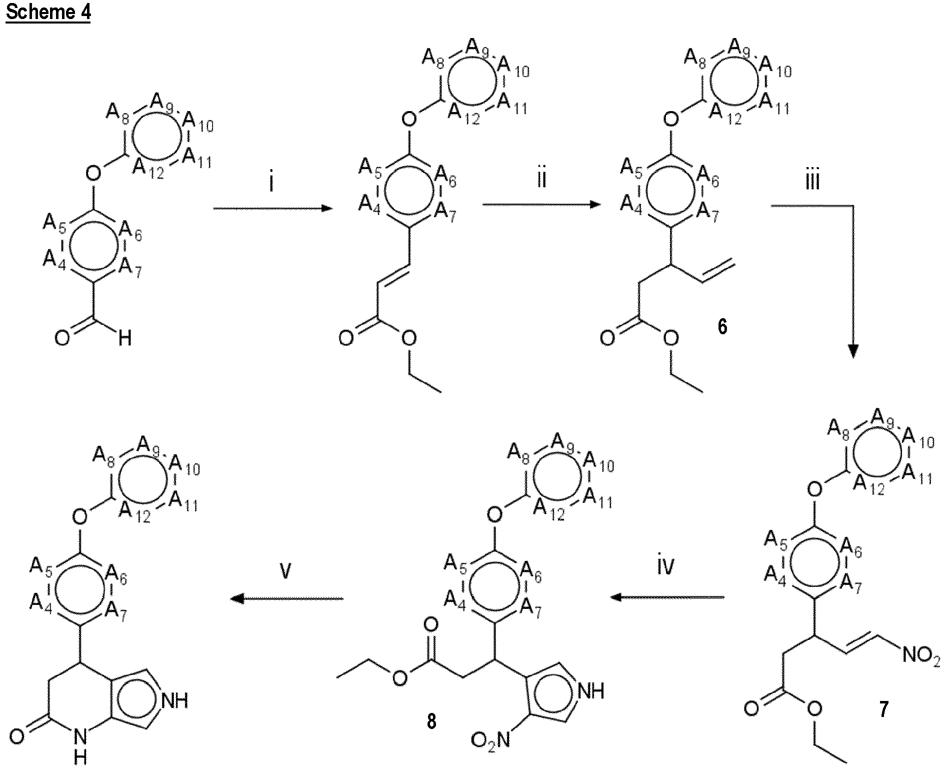

Conditions:
i) CH₃CH₂OCOCH₂P(Ph)₃Br, *t*BuOK, Et₂O;
ii) CH₂CHMgBr, PhSCu(I), THF;
iii) TEMPO, tBuONO, 1,4-dioxane;
iv) TOSMIC, tBuOK, THF; and,
v) Zn, AcOH.

Scheme **4** illustrates a general route for the formation of Formula **I** analogs wherein A₁ and A₃ are CH, A₂ is NH, Y is a single carbon-carbon bond, R₁₄ is oxygen, R₁₃ is H, each of R₁₅, R'₁₅ and R₁₆ is H and A₄, A₅, A₆, A₇, A₈, A₉, A₁₀, A₁₁ and A₁₂ have the meaning as previously described.

Intermediate **6** can be obtained via a Wittig reaction of Building block **I**, using for example CH₃CH₂OCOCH₂P(Ph)₃Br and tBuOK in Et₂O, followed by a reaction with, for instance, vinylmagnesium bromide and PhSCu(I) in THF. The subsequent introduction of the nitro group in the E-confirmation can be achieved by using, for example, TEMPO and tBuONO in 1,4-dioxane. Pyrrole intermediate **8** was obtained via a [3+2]cycloaddition of intermediate **7**, using for instance TOSMIC and tBuOK in THF. The reduction of the nitro, followed by the ring closure to obtain the Formula I analog, was performed in a single step by using, for example, zinc dust in AcOH.

### Examples

All building blocks used are commercially available, known or prepared according to methods known to those skilled in the art.

### Examples 1-86

### 1: 4-[3-methoxy-4-(3-methylphenoxy)phenyl]-2H,4H,5H,6H,7H-pyrazolo[3,4-b]pyridin-6-one

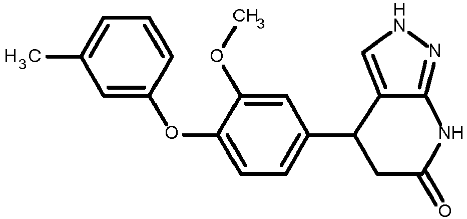

**i)** To a solution of meta-cresol (44 µL) in DMF (4 mL) were added K₂CO₃ (175) and 4-fluoro-3-methoxybenzaldehyde (65 mg). The reaction mixture was heated to 110 °C and stirred overnight. After cooling to room temperature the solvent was removed under reduced pressure. The resulting oil was partitioned between DCM and water. The water layer was extracted twice more with DCM and the combined organic layers were dried over MgSO₄, filtered and concentrated. After reversed phased flash chromatography, using a gradient from 10% to 100% ACN in water, 3-methoxy-4-(3-methvlphenoxv)benzaldehvde (66 mg) was obtained.
ii) The in the previous step obtained product (66 mg) was dissolved in 4 mL MeOH. Meldrum's acid (39 mg) and 3-aminopyrazole (23 mg) were added and the reaction was stirred 6 hours at 65 °C. The reaction was cooled to room temperature, Celite was added and the solvent was evaporated. After reversed phase chromatography, using solids loading, 4-[3-methoxy-4-(3-methylphenoxy)phenyl]-2*H*,4*H*,5*H*,6*H*,7*H*-pyrazolo[3,4-b]pyridin-6-one (25 mg) was obtained. MS(ES+) *m*/*z* 350.2 (M+H)+

Following a procedure analogous to that described for example 1, using appropriate starting materials, the following compounds have been prepared.

### 2: 4-(2-methoxy-4-{6-oxo-2H,4H,5H,6H, 7H-pyrazolo[3,4-b]pyridin-4-yl}phenoxy)-3-(trifluoromethyl)benzonitrile

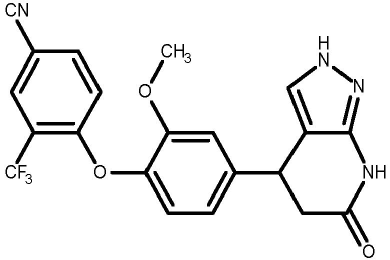

MS(ES+) *m*/*z* 429.2 (M+H)+

### 3: 4-[3-methoxy-4-(2-nitrophenoxy)phenyl]-2H,4H,5H,6H, 7H-pyrazolo[3,4-b]pyridin-6-one

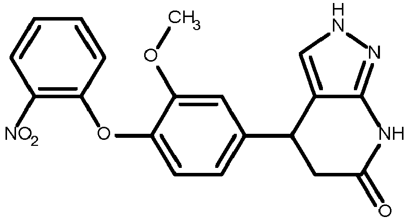

MS(ES⁺) *m*/*z* 381.2 (M+H)⁺

### 4: 4-13-methoxy-4-[2-(trifluoromethyl)phenoxy]phenyl}2H,4H,5H,6H,7H-pyrazolo[3,4-b]pyridin-6-one

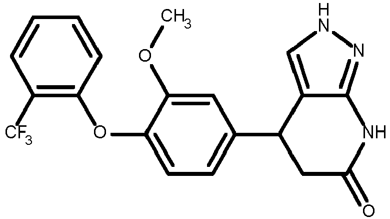

MS(ES+) *m*/*z* 404.2 (M+H)+

### 5: 4-{3-methoxy-4-[4-(trifluoromethyl)phenoxy]phenyl]2H,4H,5H,6H,7H-pyrazolo[3,4-b]pyridin-6-one

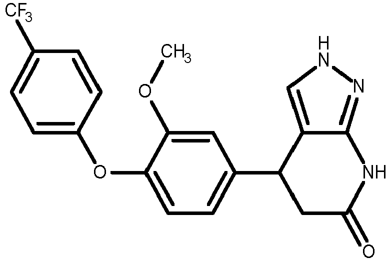

MS(ES+) *m*/*z* 404.2 (M+H)+

### 6: 4-13-methoxy-4-[3-(trifluoromethyl)phenoxy]phenyl]2H,4H,5H,6H,7H-pyrazolo[3,4-b]pyridin-6-one

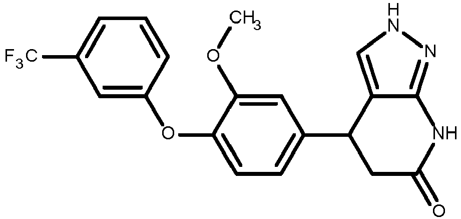

MS(ES⁺) *m*/*z* 404.2 (M+H)⁺

### 7: 4-[4-(2-bromophenoxy)-3-methoxyphenyl]-2H,4H,5H,6H, 7H-pyrazolo[3,4-b]pyridin-6-one

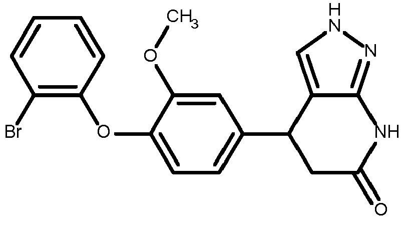

MS(ES⁺) *m*/*z* 414.2 (M+H)⁺

### 8: 4-[3-methoxy-4-(2-methylphenoxy)phenyl]-2H,4H,5H,6H, 7H-pyrazolo[3,4-b]pyridin-6-one

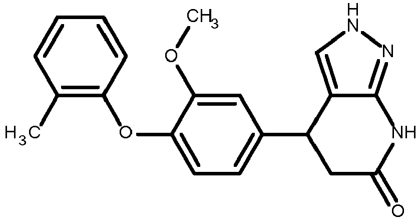

MS(ES⁺) *m*/*z* 350.2 (M+H)⁺

### 9: 2-(2-methoxy-4-{6-oxo-2H,4H,5H,6H, 7H-pyrazolo[3,4-b]pyridin-4-yl}phenoxy)benzonitrile

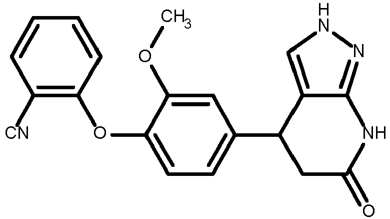

MS(ES⁺) *m*/*z* 361.2 (M+H)⁺

### 10: 4-[4-(2-tert-butylphenoxy)-3-methoxyphenyl]- 2H, 4H, 5H, 6H, 7H-pyrazolo[3,4-b]pyridin-6-one

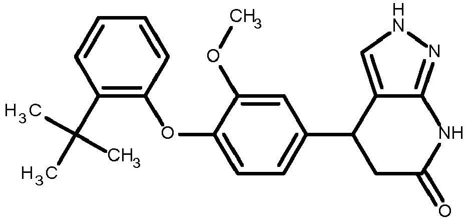

MS(ES+) *m*/*z* 392.2 (M+H)+

### 11: 4-[4-(2-chlorophenoxy)-3-methoxyphenyl]-2H,4H,5H,6H,7H-pyrazolo[3,4-b]pyridin-6-one

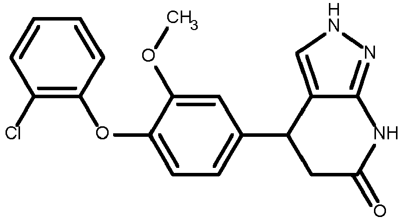

MS(ES⁺) *m*/*z* 370.2 (M+H)⁺

### 12: 4-{3-methoxy-4-[2-(trifluoromethoxy)phenoxy]phenyl}-2H,4H,5H,6H, 7H-pyrazolo[3,4-b]pyridin-6-one

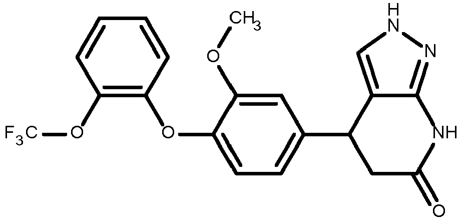

MS(ES+) *m*/*z* 420.2 (M+H)+

### 13: 4-[3-methoxy-4-(2-methoxyphenoxy)phenyl]-2H,4H,5H,6H,7H-pyrazolo[3,4-b]pyridin-6-one

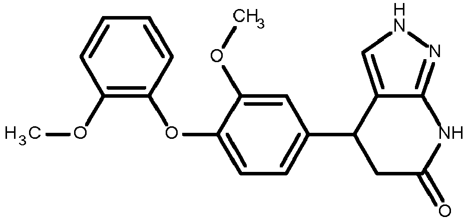

MS(ES⁺) m/z 366.2 (M+H)⁺

### 14: 4-[4-(3-bromophenoxy)-3-methoxyphenyl]-2H,4H,5H,6H,7H-pyrazolo[3,4-b]pyridin-6-one

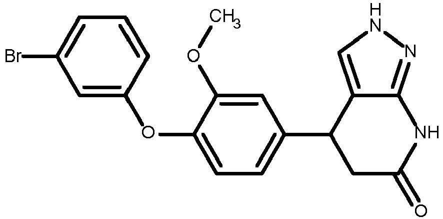

MS(ES⁺) *m*/*z* 414.2 (M+H)⁺

### 15: 4-[4-(3-tert-butylphenoxy)-3-methoxyphenyl]-2H,4H,5H,6H,7H-pyrazolo[3,4-b]pyridin-6-one

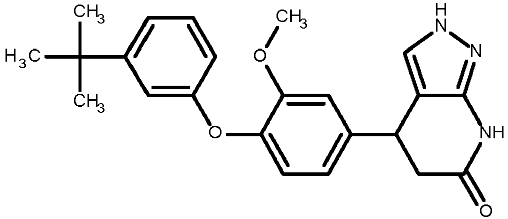

MS(ES⁺) *m*/*z* 392.2 (M+H)⁺

### 16: 4-[4-(3-chlorophenoxy)-3-methoxyphenyl]-2H,4H,5H,6H,7H-pyrazolo[3,4-b]pyridin-6-one

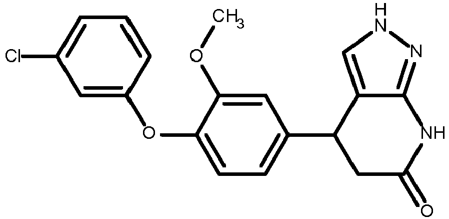

MS(ES+) *m*/*z* 370.2 (M+H)⁺

### 17: 4-{3-methoxy-4-[3-(trifluoromethoxy)phenoxy]phenyl}-2H,4H,5H,6H,7H-pyrazolo[3,4-b]pyridin-6-one

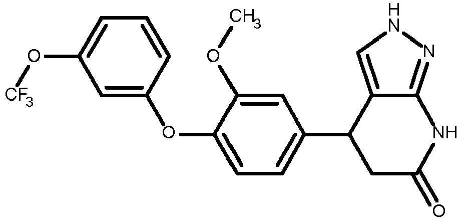

MS(ES⁺) *m*/*z* 420.2 (M+H)⁺

### 18: 4-[3-methoxy-4-(3-methoxyphenoxy)phenyl]-2H,4H,5H,6H,7H-pyrazolo[3,4-b]pyridin-6-one

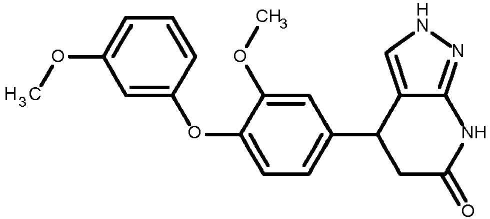

MS(ES+) *m*/*z* 366.2 (M+H)⁺

### 19: methyl 3-(2-methoxy-4-{6-oxo-2H,4H,5H,6H, 7H-pyrazolo[3,4-b]pyridin-4-yl}phenoxy)benzoate

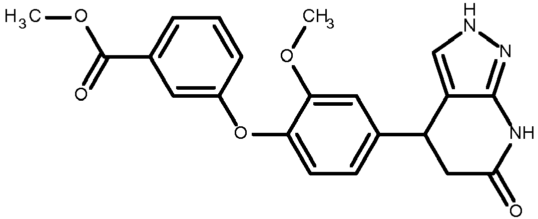

MS(ES⁺) *m*/*z* 394.2 (M+H)⁺

### 20: 4-[4-(2-ethylphenoxy)-3-methoxyphenyl]-2H,4H,5H,6H, 7H pyrazolo[3,4-b]pyridin-6-one

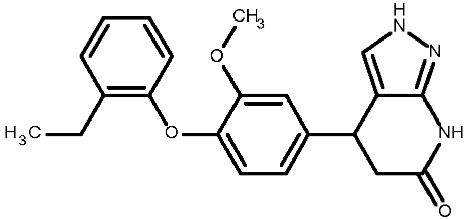

MS(ES⁺) *m*/*z* 364.2 (M+H)⁺

### 21: 4-[4-(3-ethylphenoxy)-3-methoxyphenyl]-2H,4H,5H,6H,7H-pyrazolo[3,4-b]pyridin-6-one

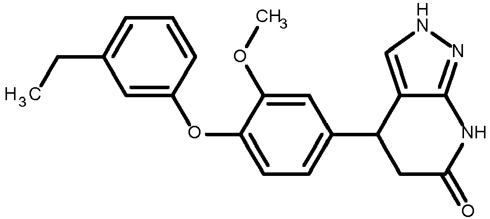

MS(ES+) *m*/*z* 364.2 (M+H)⁺

### 22: 4-[3-methoxy-4-(2-propylphenoxy)phenyl]-2H,4H,5H,6H, 7H-pyrazolo[3,4-b]pyridin-6-one

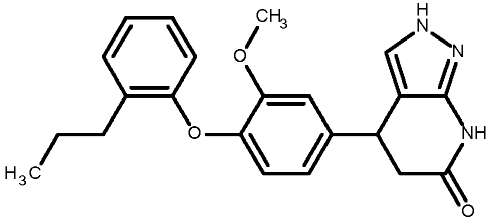

MS(ES+) *m*/*z* 378.2 (M+H)⁺

### 23: 4-{3-methoxy-4-[2-(propan-2-yl)phenoxy]phenyl}-2H,4H,5H,6H,7H-pyrazolo[3,4-b]pyridin-6-one

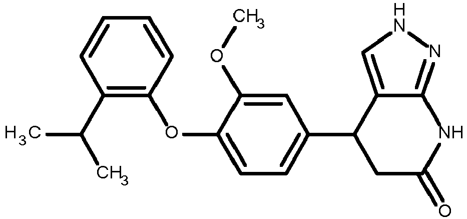

MS(ES⁺) *m*/*z* 378.2 (M+H)⁺

### 24: 4-[3-methoxy-4-(2-phenylphenoxy)phenyl]-2H,4H,5H,6H,7H-pyrazolo[3,4-b]pyridin-6-one

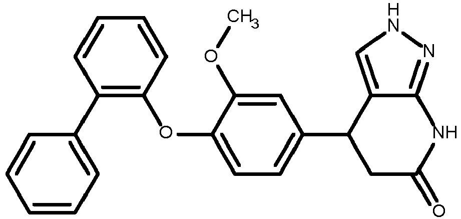

MS(ES+) *m*/*z* 412.2 (M+H)⁺

### 25: 4-[4-(2-cyclopentylphenoxy)-3-methoxyphenyl]-2H,4H,5H,6H,7H-pyrazolo[3,4-b]pyridin-6-one

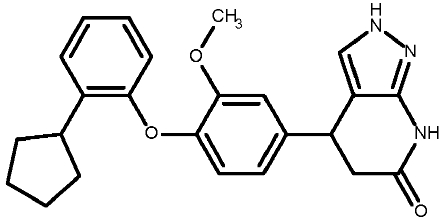

MS(ES⁺) m/z 404.2 (M+H)⁺

### 26: 4-[4-(2-benzylphenoxy)-3-methoxyphenyl]-2H,4H,5H,6H, 7H-pyrazolo[3,4-b]pyridin-6-one

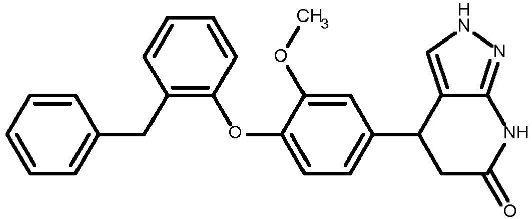

MS(ES⁺) *m*/*z* 426.2 (M+H)⁺

### 27: 4-[4-(2-tert-butyl-4-methoxyphenoxy)-3-methoxyphenyl]-2H,4H,5H,6H,7H-pyrazolo[3,4-b]pyridin-6-one

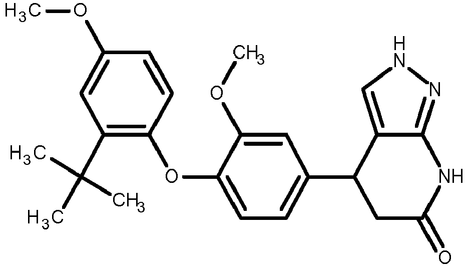

MS(ES⁺) *m*/*z* 422.2 (M+H)⁺

### 28: 4-[4-(2-cyclopropylphenoxy)-3-methoxyphenyl]-2H,4H,5H,6H,7Hpyrazolo[3,4-b]pyridin-6-one

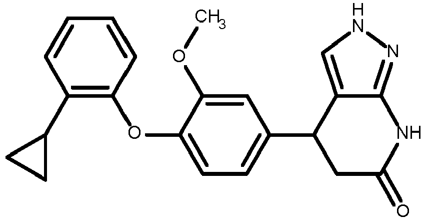

MS(ES+) m/z 376.2 (M+H)+

### 29: 4-[4-(3-tert-butyl-4-hydroxyphenoxy)-3-methoxyphenyl]-2H,4H,5H,6H,7H-pyrazolo[3,4-b]pyridin-6-one

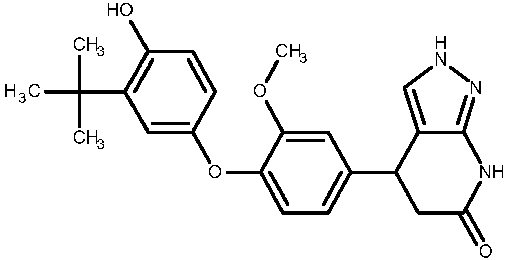

MS(ES+) *m*/*z* 408.2 (M+H)+

### 30: 4-{3-methoxy-4-[3-(propan-2-yl)phenoxy]phenyl}-2H,4H,5H,6H, 7H-pyrazolo[3,4-b]pyridin-6-one

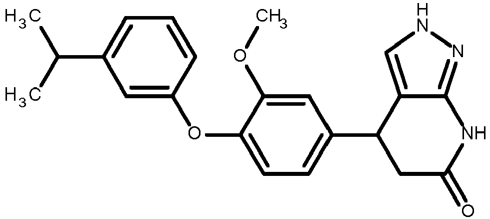

MS(ES⁺) *m*/*z* 378.2 (M+H)⁺

### 31: 4-[4-(3-cyclopropylphenoxy)-3-methoxyphenyl]-2H,4H,5H,6H,7H-pyrazolo[3,4-b]pyridin-6-one

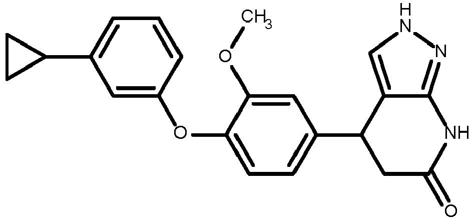

MS(ES+) *m*/*z* 376.2 (M+H)⁺

### 32: 4-[4-(2-tert-butyl-4-ethylphenoxy)-3-methoxyphenyl]-2H,4H,5H,6H,7H-pyrazolo[3,4-b]pyridin-6-one

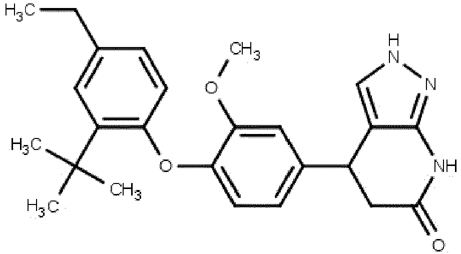

MS(ES⁺) *m*/*z* 420.2 (M+H)⁺

### 33: 4-[4-(4-bromo-2-tert-butylphenoxy)-3-methoxyphenyl]-2H,4H,5H,6H, 7H-pyrazolo[3,4-b]pyridin-6-one

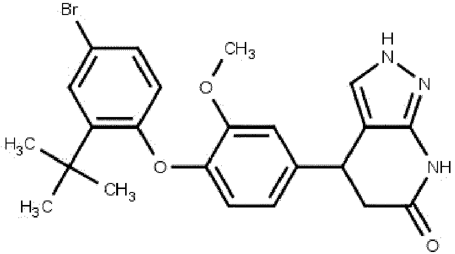

MS(ES+) *m*/*z* 470.1 (M+H)⁺

### 34: 4-[4-(2-tert-butyl-4-methylphenoxy)-3-methoxyphenyl]-2H,4H,5H,6H,7H-pyrazolo[3,4-b]pyridin-6-one

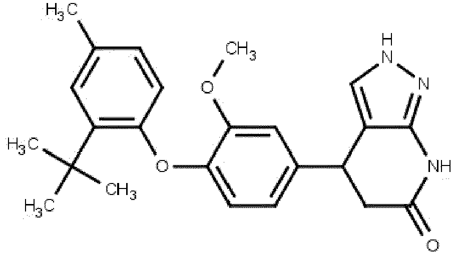

MS(ES⁺) m/z 406.2 (M+H)⁺

### 35: methyl3-tert-butyl-4-(2-methoxy-4-{6-oxo-2H,4H,5H,6H,7H-pyrazolo[3,4-b]pyridin-4-yl}phenoxy)benzoate

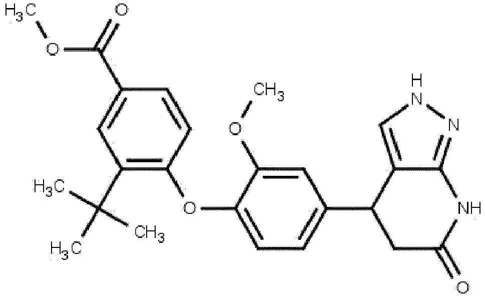

MS(ES+) *m*/*z* 450.2 (M+H)⁺

### 36: methyl 4-(2-methoxy-4-{6-oxo-2H,4H,5H,6H,7H-pyrazolo[3,4-b]pyridin-4-yl}phenoxy)naphthalene-1-carboxylate

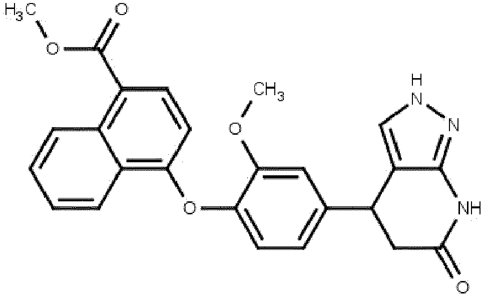

MS(ES⁺) *m*/*z* 444.1 (M+H)⁺

### 37: 4-[4-(2,4-di-tert-butylphenoxy)-3-methoxyphenyl]-2H,4H,5H,6H,7H-pyrazolo[3,4-b]pyridin-6-one

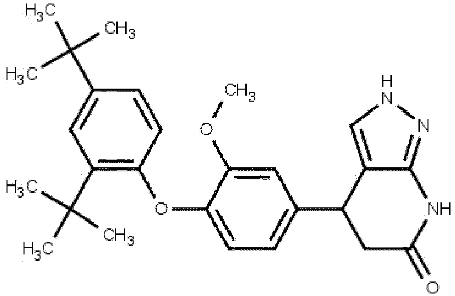

MS(ES⁺) *m*/*z* 448.3 (M+H)⁺

### 38: 4-{3-methoxy-4-[3-methoxy-5-(trifluoromethyl)phenoxy]phenyl}-2H,4H,5H,6H,7H-pyrazolo[3,4-b]pyridin-6-one

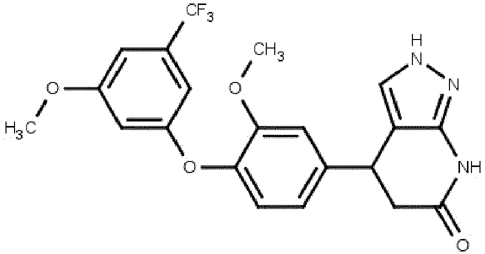

MS(ES+) *m*/*z* 434.1 (M+H)+

### 39: 3-tert-butyl-4-(2-methoxy-4-{6-oxo-2H,4H,5H,6H, 7H-pyrazolo[3,4-b]pyridin-4-yl}phenoxy)benzonitrile

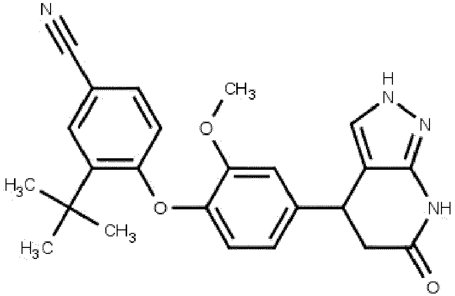

MS(ES⁺) *m*/*z* 417.2 (M+H)⁺

### 40: 4-{3-methoxy-4-[2-(pentafluoro-λ⁶-sulfanyl)phenoxy]phenyl}-2H,4H,5H,6H,7H-pyrazolo[3,4-b]pyridin-6-one

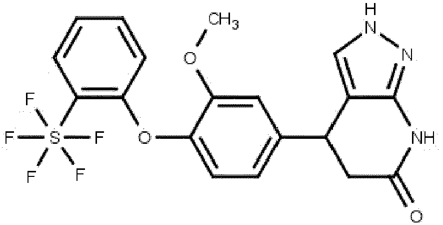

MS(ES⁺) *m*/*z* 462.1 (M+H)⁺

### 41: methyl 3-(2-methoxy-4-{6-oxo-2H,4H,5H,6H, 7H-pyrazolo[3,4-b]pyridin-4-yl}phenoxy)-5-(trifluoromethyl)benzoate

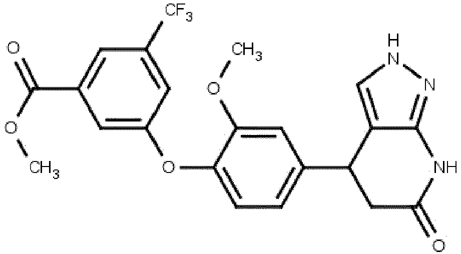

MS(ES+) *m*/*z* 462.1 (M+H)⁺

### 42: 4-{4-[3-bromo-5-(trifluoromethyl)phenoxy]-3-methoxyphenyl}-2H,4H,5H,6H,7H-pyrazolo[3,4-b]pyridin-6-one

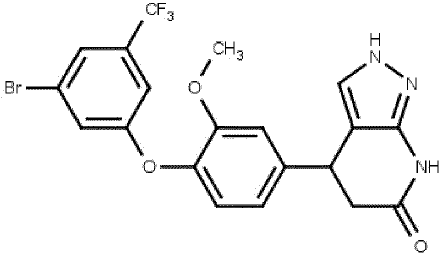

MS(ES⁺) *m*/*z* 482.0 (M+H)⁺

### 43: 4-{4-[3,5-bis(trifluoromethyl)phenoxy]-3-methoxyphenyl}-2H,4H,5H,6H,7H-pyrazolo[3,4-b]pyridin-6-one

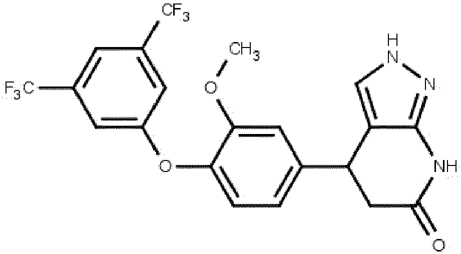

MS(ES+) *m*/*z* 472.1 (M+H)⁺

### 44: 4-[4-(3,5-di-tert-butylphenoxy)-3-methoxyphenyl]-2H,4H,5H,6H,7H-pyrazolo[3,4-b]pyridin-6-one

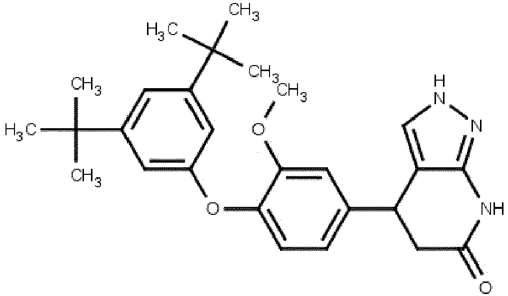

MS(ES+) *m*/*z* 448.3 (M+H)⁺

### 45: 4-[4-(2-{bicyclo[1.1.1]pentan-1-yl}phenoxy)-3-methoxyphenyl]-2H,4H,5H,6H, 7H pyrazolo[3,4-b]pyridin-6-one

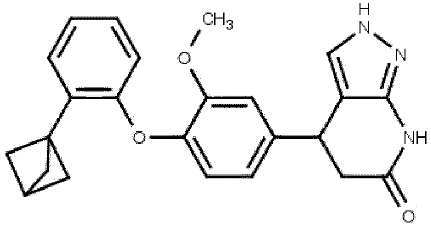

MS(ES⁺) *m*/*z* 402.2 (M+H)⁺

### 46: 4-(2-methoxy-4-{6-oxo-2H,4H,5H,6H,7H-pyrazolo[3,4-b]pyridin-4-yl}phenoxy)naphthalene-1-carbonitrile

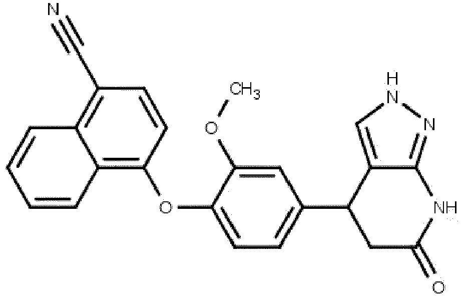

MS(ES⁺) *m*/*z* 411.1 (M+H)⁺

### 47: 4-[4-(2,3-dihydro-1H-inden-4-yloxy)-3-methoxyphenyl]-2H,4H,5H,6H,7H-pyrazolo[3,4-b]pyridin-6-one

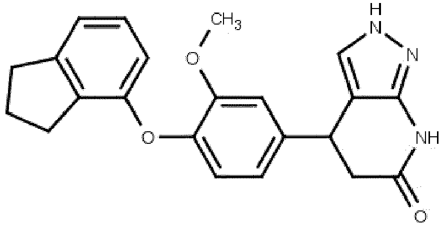

MS(ES+) *m*/*z* 376.2 (M+H)⁺

### 48: 4-{4-[(2,2-dimethyl-2,3-dihydro-1-benzofuran-7-yl)oxy]-3-methoxyphenyl}-2H,4H,5H,6H, 7H pyrazolo[3,4-b]pyridin-6-one

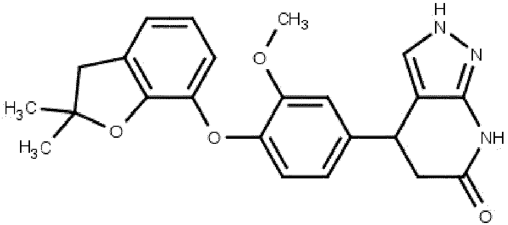

MS(ES+) *m*/*z* 406.2 (M+H)⁺

### 49: 4-[4-(2,3-dihydro-1H-inden-5-yloxy)-3-methoxyphenyl]-2H,4H,5H,6H,7H-pyrazolo[3,4-b]pyridin-6-one

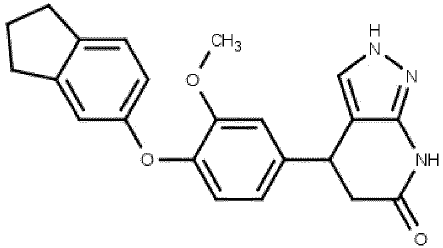

MS(ES⁺) *m*/*z* 376.2 (M+H)⁺

### 50: methyl 8-(2- methoxy-4-{6-oxo- 2H, 4H, 5H, 6H, 7H-pyrazolo[3,4-b]pyridin-4-yl}phenoxy)quinoline-5-carboxylate

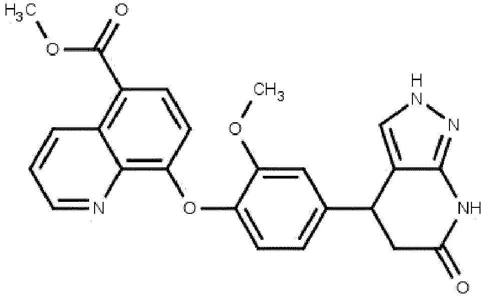

MS(ES+) *m*/*z* 445.1 (M+H)⁺

### 51: 4-(3-methoxy-4-phenoxyphenyl)-2H,4H,5H,6H, 7H-pyrazolo[3,4-b]pyridin-6-one

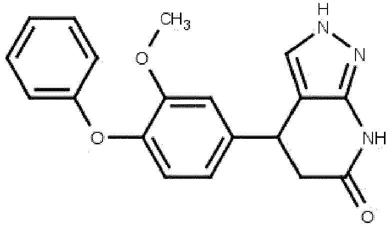

MS(ES⁺) *m*/*z* 336.1 (M+H)⁺

### 52: 4-{3-bromo-4-[3-methoxy-5-(trifluoromethyl)phenoxy]phenyl}-2H,4H,5H,6H,7H-pyrazolo[3,4-b]pyridin-6-one

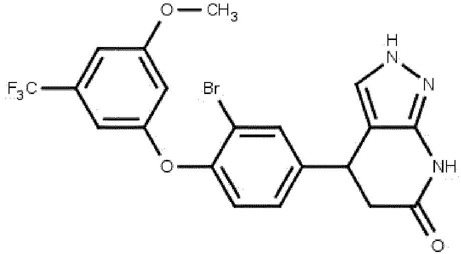

MS(ES⁺) *m*/*z* 482.0 (M+H)⁺

### 53: 3-tert-butyl-4-(4-16-oxo-2H,4H,5H,6H,7H-pyrazolo[3,4-b]pyridin-4-yl}phenoxy)benzonitrile

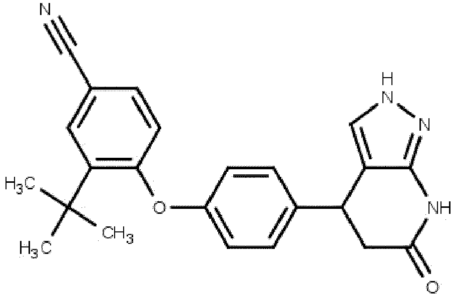

MS(ES⁺) *m*/*z* 387.2 (M+H)⁺

### 54: 4-{4-[3-methoxy-5-(trifluoromethyl)phenoxy]phenyl}-2H,4H,5H,6H,7H-pyrazolo[3,4-b]pyridin-6-one

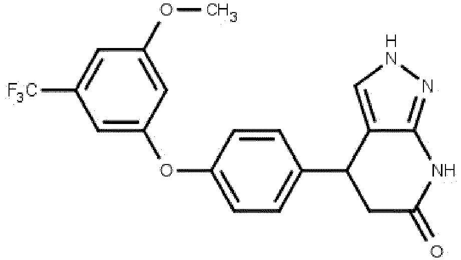

MS(ES⁺) *m*/*z* 404.1 (M+H)⁺

### 55: 3-tert-butyl-4-(2-methyl-4-{6-oxo-2H,4H,5H,6H,7H-pyrazolo[3,4-b]pyridin-4-yl}phenoxy)benzonitrile

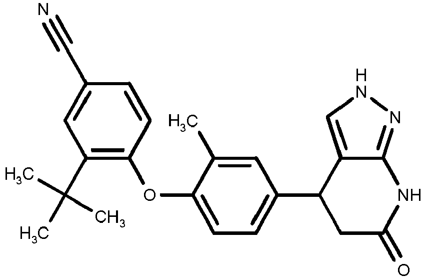

MS(ES⁺) *m*/*z* 400.2 (M+H)⁺

### 56: 4-14-[3-methoxy-5-(trifluoromethyl)phenoxy]-3-methylphenyl]-2H,4H,5H,6H,7H-pyrazolo[3,4-b]pyridin-6-one

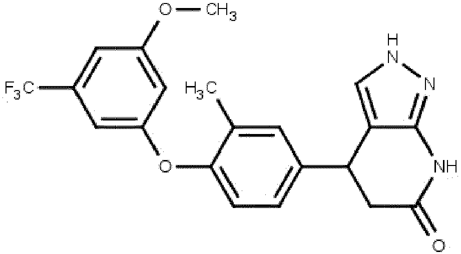

MS(ES⁺) m/z 418.1 (M+H)⁺

### 57: 4-{3-fluoro-4-[3-methoxy-5-(trifluoromethyl)phenoxy]phenyl}-2H,4H,5H,6H,7H-pyrazolo[3,4-b]pyridin-6-one

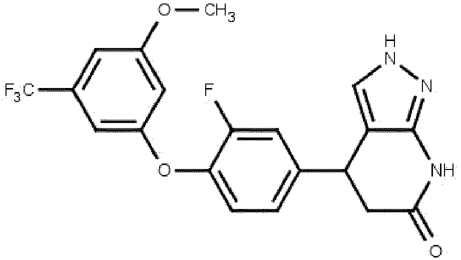

MS(ES+) *m*/*z* 422.1 (M+H)⁺

### 58: 4-{4-[2-(pentafluoro-λ⁶-sulfanyl)phenoxy]-3-(trifluoromethoxy)phenyl}-2H,4H,5H,6H, 7H-pyrazolo[3,4-b]pyridin-6-one

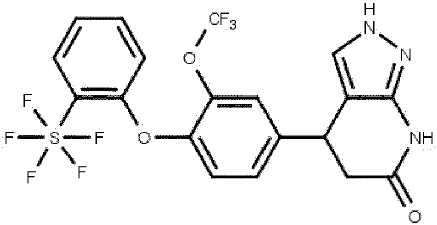

MS(ES+) *m*/*z* 516.1 (M+H)⁺

### 59: 4-{4-[2-(pentafluoro-λ⁶-sulfanyl)phenoxy]phenyl}-2H,4H,5H,6H,7H-pyrazolo[3,4-b]pyridin-6-one

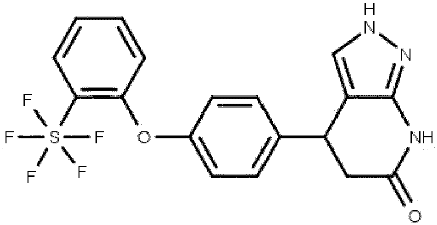

MS(ES⁺) *m*/*z* 432.1 (M+H)⁺

### 60: 4-[3-(trifluoromethoxy)-4-[2-(trifluoromethyl)phenoxy]phenyl]-2H,4H,5H,6H,7H-pyrazolo[3,4-b]pyridin-6-one

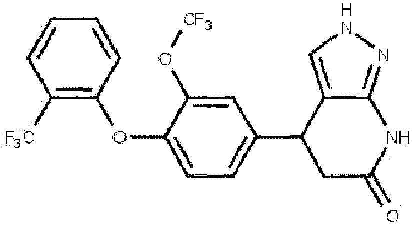

MS(ES⁺) *m*/*z* 458.1 (M+H)⁺

### 61: 3-tert-butyl-4-(2-methoxy-4-{6-oxo-2H,4H,5H,6H,7H-pyrazolo[3,4-b]pyridin-4-yl}phenoxy)benzoic acid

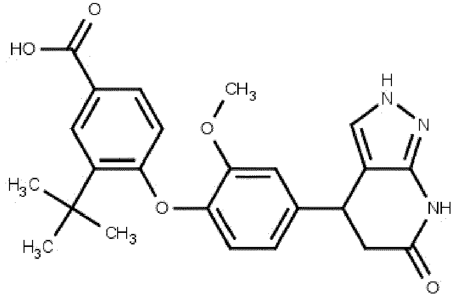

MS(ES⁺) *m*/*z* 436.2 (M+H)⁺

### 62: 3-(2-methoxy-4-{6-oxo-2H,4H,5H,6H,7H-pyrazolo[3,4-b]pyridin-4-yl}phenoxy)-5-(trifluoromethyl)benzoic acid

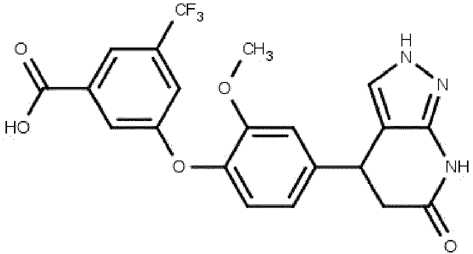

MS(ES⁺) *m*/*z* 448.1 (M+H)⁺

### 63: 4-{3-methoxy-4-[3-methyl-5-(trifluoromethyl)phenoxy]phenyl}-2H,4H,5H,6H, 7H-pyrazolo[3,4-b]pyridin-6-one

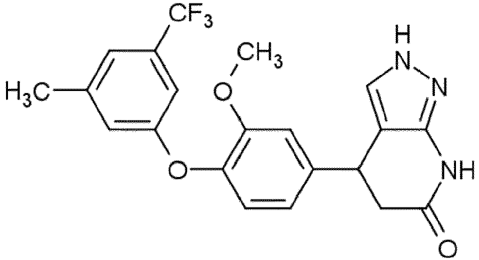

MS(ES⁺) *m*/*z* 418.4 (M+H)⁺

### 64: 4-[4-(3,5-dimethylphenoxy)-3-methoxyphenyl]-2H,4H,5H,6H, 7H-pyrazolo[3,4-b]pyridin-6-one

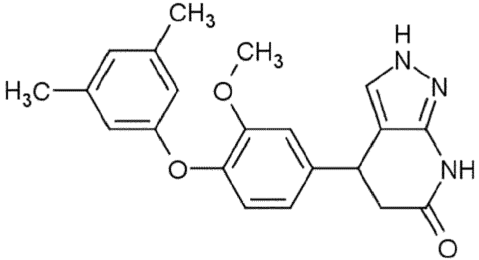

MS(ES⁺) *m*/*z* 364.4 (M+H)⁺

### 65: 4-{3-methoxy-4-[2-methyl-4-(trifluoromethyl)phenoxy]phenyl}-2H,4H,5H,6H, 7H-pyrazolo[3,4-b]pyridin-6-one

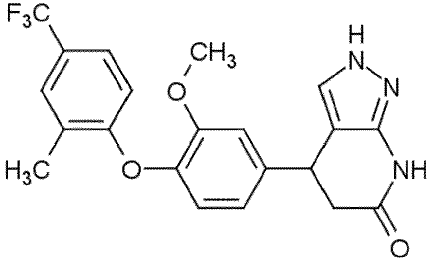

MS(ES⁺) *m*/*z* 418.4 (M+H)⁺

### 66: 4-[4-(2,4-dimethylphenoxy)-3-methoxyphenyl]-2H,4H,5H,6H, 7H-pyrazolo[3,4-b]pyridin-6-one

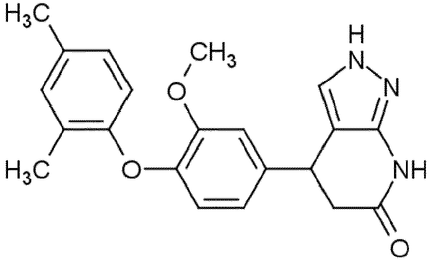

MS(ES+) *m*/*z* 364.4 (M+H)⁺

### 67: 4-[4-(2-tert-butyl-4-ethylphenoxy)-3-methoxyphenyl]-2H, 6H,7H-pyrazolo[3,4-b]pyridin-6-one

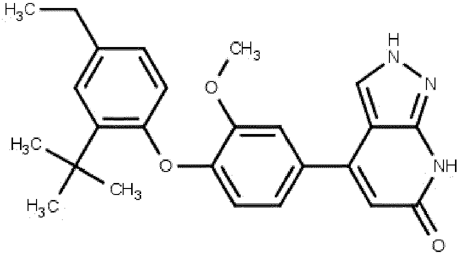

**i)** Example 32 (32 mg) was dissolved in 1.5 mL 1,4-dioxane and DDQ (17 mg) was added. The reaction was heated to 50 °C and stirred for 1 hour. The solvent was evaporated and sat. aq. NaHCOs was added to give a suspension. The suspension was sonicated and the solids were filtered off and washed with water three times. The solids were dried in the vacuum oven to afford 4-[4-(2-*tert*-butyl-4-ethylphenoxy)-3-methoxypheny]-*2H,6H,7H*-pyrazolo[3,4-*b*]pyridin-6-one (23 mg). MS(ES+) *m*/*z* 418.2 (M+H)⁺

Following a procedure analogous to that described for **Example 67,** using appropriate starting materials, the following compounds have been prepared.

### 68: 4-[4-(2-tert-butyl-4-methoxyphenoxy)-3-methoxyphenyl]-2H,6H,7H-pyrazolo[3,4-b]pyridin-6-one

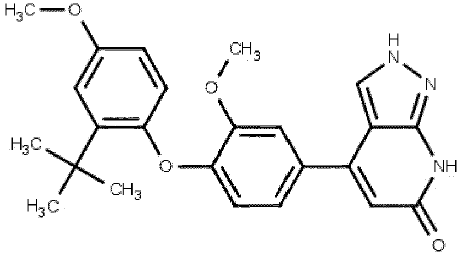

MS(ES+) *m*/*z* 420.2 (M+H)⁺

### 69: 4-[4-(2-tert-butyl-4-methylphenoxy)-3-methoxyphenyl]-2H,6H, 7H pyrazolo[3,4-b]pyridin-6-one

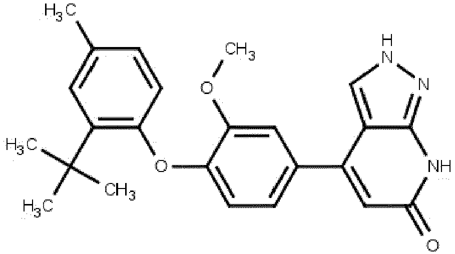

MS(ES⁺) *m*/*z* 404.2 (M+H)⁺

### 70: 4-[4-(4-bromo-2-tert-butylphenoxy)-3-methoxyphenyl]-2H,6H,7H-pyrazolo[3,4-b]pyridin-6-one

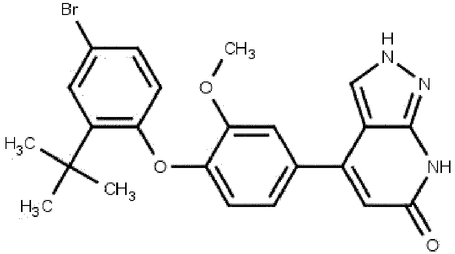

MS(ES⁺) *m*/*z* 468.1 (M+H)⁺

### 71: 4-(2-methoxy-4-{6-oxo-2H,6H, 7H-pyrazolo[3,4-b]pyridin-4-yl}phenoxy)-3-(trifluoromethyl)benzonitrile

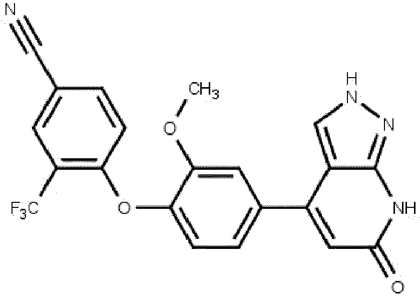

MS(ES⁺) *m*/*z* 427.1 (M+H)⁺

### 72: methyl 4-{4-[3,5-bis(trifluoromethyl)phenoxy]-3-methoxyphenyl}-6-oxo-2H,4H,5H,6H,7H-pyrazolo[3,4-b]pyridine -5-carboxylate

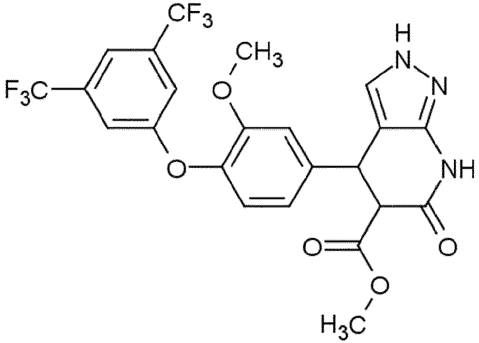

**i)** Following a procedure analogous to that described in **Example 1, step i),** using appropriate starting materials, 4-13,5-bis(trifluoromethyl)phenoxy]-3-methoxybenzaldehyde was obtained.
**ii**) The in the previous step obtained product (211 mg), Meldrum's acid (85 mg) and 3-aminopyrazole (48 mg) were dissolved in MeOH (5 mL). The reaction mixture was stirred for 3.5 hours at room temperature and the solvent was evaporated by a flow of nitrogen. This resulted in 4-{4-[3,5-bis(trifluoromethyl)phenoxy]-3-methoxyphenyl}-6-oxo-2H,4H,5H,6H,7H-pyrazolo[3,4-b]pyridine-5-carboxylic acid (200 mg).
**iv**) DIAD (23 µL) was added dropwise under a nitrogen atmosphere to a solution of PPh₃ (31.4 mg), MeOH (40 µL) and the in the previous step obtained product (50 mg) in anhydrous THF (1 mL). The reaction was stirred overnight at room temperature. Water was added and the product was extracted with DCM three times. The combined organic layers were dried over MgSO₄ and concentrated. The crude product was purified by reversed phase chromatography, using a gradient of 40 to 75% ACN in water (containing 0.1% TFA), to obtain methyl 4-{4-[3,5-bis(trifluoromethyl)phenoxy1-3-methoxyphenyl}-6-oxo-2H,4H,5H,6H,7H-pyrazolo[3,4-b]pyridine-5-carboxylate (16.6 mg). MS(ES+) *m*/*z* 530.4 (M+H)+

Following a procedure analogous to that described for **Example 72,** using appropriate starting materials, the following compound has been prepared.

### 73: ethyl 4-{4-[3,5-bis(trifluoromethyl)phenoxy]-3-methoxyphenyl}-6-oxo-2H,4H,5H,6H,7H-pyrazolo[3,4-b]pyridine -5-carboxylate

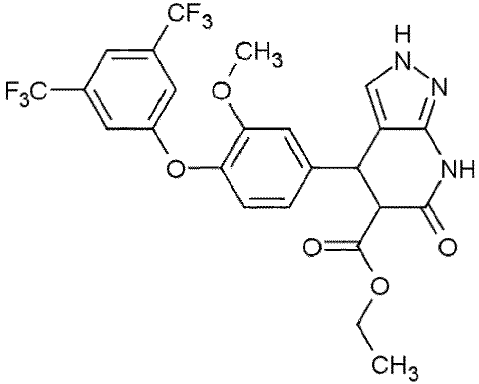

MS(ES+) *m*/*z* 544.4 (M+H)⁺

### 74: 4-{4-[3,5-bis(trifluoromethyl)phenoxy]-3-methoxyphenyl}-N,N-dimethyl-6-oxo-2H,4H,5H,6H,7H-pyrazolo[3,4-b]pyridine-5-carboxamide

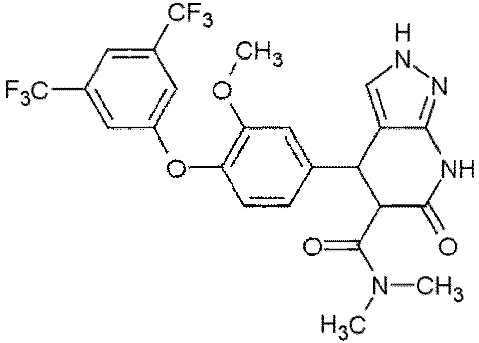

**i)** The product obtained in **Example 72, step ii)** (50 mg), DMAP (20 mg) and dimethylamine HCl (12 mg) were dissolved in DCM (1 mL). EDC (30 mg) was added and the reaction was stirred at room temperature overnight. Water was added and the product was extracted with DCM three times. The combined organic layers were dried over MgSO₄ and concentrated. The crude product was purified by reversed phase chromatography, using a gradient of 40 to 75% ACN in water (containing 0.1% TFA), to obtain 4-{4-[3,5-bis(trifluoromethyl)phenoxyl-3-methoxyphenyl]-N,N-dimethyl-6-oxo-2H,4H,5H,6H,7H-pvrazolo[3,4-b]pyridine-5-carboxamide (9.3 mg). MS(ES+) *m*/*z* 543.4 (M+H)⁺

Following a procedure analogous to that described for **Example 74,** using appropriate starting materials, the following compound has been prepared.

### 75: 4-{4-[3,5-bis(trifluoromethyl)phenoxy]-3-methoxyphenyl}-N,N-diethyl-6-oxo-2H,4H,5H,6H,7H-pyrazolo[3,4-b]pyridine-5-carboxamide

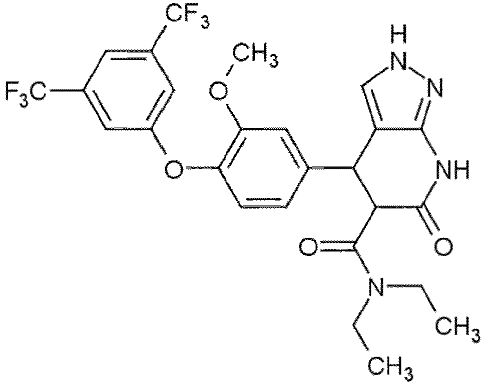

MS(ES⁺) *m*/*z* 571.5 (M+H)⁺

### 76: 4-{4-[3,5-bis(trifluoromethyl)phenoxy]-3-methoxyphenyl}-1H,2H,3H,4H,6H-pyrrolo[3,4-b]pyridin-2-one

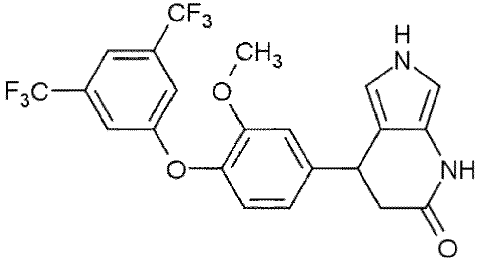

**i)** Following a procedure analogous to that described in **Example 1, step i),** using appropriate starting materials, 4-[3,5-bis(trifluoromethvl)phenoxyl-3-methoxvbenzaldehyde was obtained.
**ii**) Ethoxycarbonylmethyl)triphenylphosphonium bromide (3.54 g) was added to a suspension of tBuOK (1.03 g) in Et₂O (30 mL). The resulting mixture stirred at 50 °C for 30 minutes and then cooled to room temperature. The in the previous step obtained compound (1.50 g) dissolved in Et₂O (10 mL) was added to the reaction mixture. The reaction was stirred overnight, then quenched with water and the aqueous layer was extracted with Et₂O three times. The combined organic layers were dried over MgSO₄ and concentrated under reduced pressure. The crude product was purified on silica column, using a gradient of 0 to 20% EtOAc in heptane, to obtain ethyl (*E*)-3-{4-[3,5-bis(trifluoromethyl)phenoxy1-3-methoxyphenyl}prop-2-enoate (1.42 g).
**iii**) Vinylmagnesium bromide (1.0 M in THF, 9.67 mL) was added dropwise to a suspension of phenylsulfanylcopper (558 mg) in anhydrous THF (15 mL) at -40 °C under a nitrogen atmosphere. The reaction was allowed to reach -25 °C and again cooled down to -40 °C. The in the previous step obtained compound (1.4 g) in anhydrous THF (15 mL) was added dropwise and the reaction was stirred for 30 minutes at -40 °C. The reaction was quenched by pouring the mixture into sat. aq. NH₄Cl. The obtained solids were filtered off and the filtrate was extracted with EtOAc twice. The combined organic layers were dried over MgSO₄ and concentrated under reduced pressure. The crude product was purified on silica column, using a gradient of 0 to 30% EtOAc in heptane, to obtain ethyl 3-{4-[3,5-bis(trifluoromethyl) phenoxy]-3-methoxyphenyl}pent-4-enoate (279 mg).
**iv)** The in the previous step obtained product (275 mg), TEMPO (38 mg) and tBuONO (157 uL) were dissolved in 1,4-dioxane (5 mL), heated to 80 °C and stirred overnight. Water was added to the reaction and the aqueous layer was extracted with DCM three times. The combined organic layers were dried over MgSO₄ and concentrated under reduced pressure. The crude product was purified on silica column, using a gradient of 0 to 30% EtOAc in heptane, to obtain ethyl (*E*)-3-(4-[3,5-bis(trifluoromethvl)phenoxy1-3-methoxyphenyl}-5-nitropent-4-enoate (95 mg).
**v**) TOSMIC (37 mg) was added to a solution of tBuOK (42 mg) in anhydrous THF (1 mL) at -78 °C and stirred for 10 minutes. The compound obtained from the previous step (95 mg) was dissolved in anhydrous THF (4 mL) and added dropwise to the reaction mixture. After 10 minutes stirring at -78 °C the reaction was poured into sat. aq. NH₄Cl and the aqueous layer was extracted with EtOAc three times. The combined organic layers were dried over MgSO₄ and concentrated under reduced pressure. The crude product was purified on silica column, using a gradient of 0 to 70% EtOAc in heptane, to obtain ethyl 3-{4-[3,5-bis(trifluoromethyl)phenoxy]-3-methoxyphenyl}-3-(4-nitro-1H-pyrrol-3-yl)propanoate (48 mg).
**vi**) To a solution of the in the previous step obtained compound (45 mg) in AcOH (1.5 mL) was added zinc dust (54 mg). The reaction was heated to 50 °C and stirred for 30 minutes. The reaction mixture was filtered over celite and the filter cake was rinsed with EtOAc. The filtrate was washed with sat. aq. NaHCOs and water and then dried over MgSO₄ and concentrated under reduced pressure. The crude product was purified by reversed phase chromatography, using a gradient of 40 to 90% ACN in water, to obtain 4-{4-[3,5-bis(trifluoromethyl)phenoxyl-3-methoxyphenyl}-*1H,2H,3H,4H,6H*-pyrrolo[3,4-*b*]pyridin-2-one (8.7 mg). MS(ES+) *m*/*z* 471.4 (M+H)⁺

### 77: 4-{4-[3-(difluoromethyl)-5-(trifluoromethyl)phenoxy]-3-methoxyphenyl}-2H,4H,5H,6H,7H-pyrazolo[3,4-b]pyridin-6-one

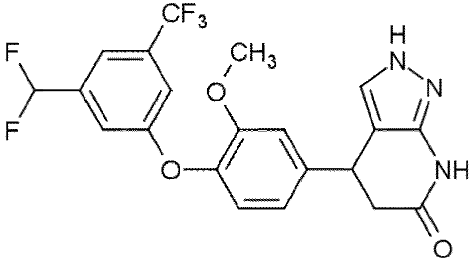

**i)** 4-Fluoro-3-methoxy-benzonitrile (250 mg), 3-hydroxy-5-(trifluoromethyl)benzaldehyde (0.23 mL) and K₂CO₃ (457 mg) suspended in DMF (10mL). The reaction was heated to 90°C and stirred overnight. The reaction was cooled to room temperature and partitioned between water and EtOAc. The water layer was extracted twice more with EtOAc and the combined organic layers were washed with brine, dried over MgSO₄ and concentrated. The crude product was purified by reversed phase chromatography, using a gradient of 10 to 100% ACN in water, to obtain 4-[3-formyl-5-(trifluoromethyl)phenoxy1-3-methoxy-benzonitrile (46 mg).
**ii**) The in the previous step obtained product (96 mg) was dissolved in DCM (6 mL) under a nitrogen atmosphere. DAST (0.14 mL) was added dropwise and the reaction was stirred overnight at room temperature. The reaction was placed on a water bath and sat. aq. NaHCOs (15 mL) was added. The mixture was stirred vigorously for 10 min. The aqueous layer was extracted with DCM twice. The combined organic layers were dried over MgSO₄ and concentrated to afford 4-[3-(difluoromethyl)-5-(trifluoromethyl)phenoxyl-3-methoxy-benzonitrile (102 mg).
**iii**) The in the previous step obtained compound (101 mg) was dissolved in toluene (4.5 mL) under a nitrogen atmosphere and cooled to 0 °C. A DiBAI-H solution 1.2M in toluene (0.37 mL) was added dropwise and the reaction was stirred for 1.5 hours. Aqueous HCl 6N (1.5 mL) was added and the mixture was stirred until the bubbling stopped. The mixture was warmed to room temperature and partitioned between EtOAc and half sat. aq. NaHCOs. The aqueous layer was extracted twice more with EtOAc and the combined organic layers were washed with brine, dried over MgSO₄ and concentrated. The crude product was purified on silica column, using a gradient of 0 to 25% EtOAc in heptane, to obtain 4-f3-(difluoromethyl)-5-(trifluoromethyl)phenoxyl-3-methoxy-benzaldehyde (71 mg).
**iv**) Analogous to the procedure described in **Example 1, step ii),** the compound obtained in the previous step was converted into 4-{4-[3-(difluoromethyl)-5-(trifluoromethyl)phenoxy]-3-methoxyphenyl}-*2H,4H,5H,6H,7H-*pyrazolo[3,4-*b*]pyridin-6-one (28 mg). MS(ES+) *m*/*z* 454.4 (M+H)⁺

### 78: 4-{4-[2-(difluoromethyl)-4-(trifluoromethyl)phenoxy]-3-methoxyphenyl}-2H,4H,5H,6H,7H-pyrazolo[3,4-b]pyridin-6-one

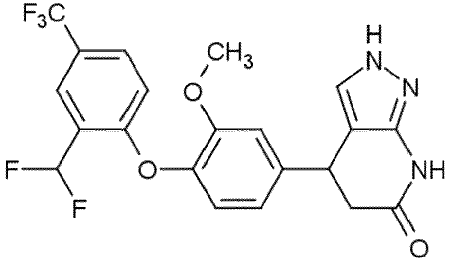

**i)** 4-Hydroxy-3-methoxybenzonitrile (250 mg), 2-fluoro-5-(trifluoromethyl)benzaldehyde (322 mg) and K₂CO₃ (463 mg) were suspended in DMF (8mL) and stirred at 90°C for 2,5 hours. The reaction was cooled to room temperature and partitioned between water and EtOAc. The water layer was extracted twice more with EtOAc and the combined organic layers were washed with brine, dried over MgSO₄ and concentrated. The crude product was purified on silica column, using a gradient of 0 to 30% EtOAc in heptane, to obtain 4-[2-formyl-4-(trifluoromethyl)phenoxyl-3-methoxy-benzonitrile (402 mg).
**ii)** Analogous to the procedures described in **Example 77, step ii)** to **step iv),** the compound obtained in the previous step was converted to 4-{4-[2-(difluoromethyl)-4-(trifluoromethyl)phenoxyl-3-methoxyphenyl]-*2H,4H,5H,6H,7H-*pyrazolo[3,4-*b*]pvridin-6-one (78 mg). MS(ES+) *m*/*z* 454.4 (M+H)⁺

The single enantiomers of **Example 43** can be obtained by chiral separation. 2 g of racemic **Example 43** was dissolved in EtOH (50 mg/mL). The solution was injected numerous times on the chiral SFC using a preparative IG column (available from Daicel, amylose tris(3-chloro-5-methylphenylcarbamate) chiral stationary phase) and an isocratic gradient of 30% EtOH and 70% liquid CO₂, to obtain: 0.9 g of the (+)enantiomer **(Example 79**) at an Enantiomeric Excess of 99.6%; and, 0.9 g of the (-)enantiomer **(Example 80)** at an Enantiomeric Excess 99.0%. The optical purity of the enantiomers was determined by chiral HPLC.

The absolute configuration of the compounds of Examples 79 to 86 is not known. These compounds were dissolved in DMSO (10mg/mL) and characterized by their optical rotation, using a Jasco P-2000 Polarimeter having the following configuration: Tungsten-Halogen (WI) light source; Glan-Taylor Prism Polarizer; Quartz Faraday Cell; and, a monitor wavelength of 589 nm.
79: (+)-4-{4-[3,5-bis(trifluoromethyl)phenoxy]-3-methoxyphenyl}-2H,4H,5H,6H, 7H-pyrazolo[3,4-b]pyridin-6-one
**80**: (-)-4-14-[3,5-bis(trifluoromethyl)phenoxy]-3-methoxyphenyll-2H,4H,5H,6H, 7H-pyrazolo[3,4-b]pyridin-6-one

Following a procedure analogous to that described for **Examples 79** and **80,** using appropriate starting materials and HPLC method, the following compounds have been prepared.
**81**: (+)-4-[4-(3,5-dimethylphenoxy)-3-methoxyphenyl]-*2H,4H,5H,6H,7H*-pyrazolo[3,4-*b*]pyridin-6-one
**82**: (-)-4-[4-(3,5-dimethylphenoxy)-3-methoxyphenyl]-2H,4H,5H,6H, 7H-pyrazolo[3,4-b]pyridin-6-one
**83**: (+)-4-{3-methoxy-4-[3-methyl-5-(trifluoromethyl)phenoxy]phenyl}-*2H,4H,5H,6H,7H*-pyrazolo[3,4-*b*]pyridin-6-one
**84**: (-)-4-{3-methoxy-4-[3-methyl-5-(trifluoromethyl)phenoxy]phenyl}-2H,4H,5H,6H, 7H-pyrazolo[3,4-b]pyridin-6-one
**85**: (+)-4-14-[3-(difluoromethyl)-5-(trifluoromethyl)phenoxy]-3-methoxyphenyll-2H,4H,5H,6H, 7H-pyrazolo[3,4-b]pyridin-6-one
**86**: (-)-4-14-[3-(difluoromethyl)-5-(trifluoromethyl)phenoxy]-3-methoxyphenyll-2H,4H,5H,6H, 7H-pyrazolo[3,4-b]pyridin-6-one

### Example 87

### ERRα AlphaScreen Assay

This assay was based on the knowledge that nuclear receptors interact with cofactors in a ligand dependent matter. The sites of interaction have been mapped to LXXLL motifs that are present in the co-activator sequences. Short peptide sequences that contain the LXXLL motif mimic the behavior of full length co-activators.

The ERRα AlphaScreen Assay described here relies on the interaction of the co-activator peptide with purified bacterial-expressed ERRα ligand binding domain (ERRα-LBD); upon ligand binding, the ERRα protein can undergo a conformational change resulting in a loss of co-activator binding.

Compounds of the present invention were tested for their ability to disrupt binding of ERRα-LBD protein to co-activator peptide using AlphaScreen^{®} Technology (Perkin Elmer). ERRα-LBD protein was expressed in E. coli as a 6xHis Small Ubiquitin-like Modifier (SUMO) fusion. Bacterial expressed 6His-SUMO-ERRα-LBD protein was purified using affinity chromatography. All experiments were performed at room temperature in 384-well white non-binding plates (Greiner) using 50 mM Tris-HCl pH 7.5, 100 mM NaCl, 0,1% Pluronic F-127, 0.05% BSA and 5 mM TCEP as the buffer. Final DMSO concentration was 1% in the assay. Compounds were assayed in triplicate and: incubated with 0.81 nM ERRα-LBD protein and 10 µg/mL streptavidin donor beads and 10 µg/mL Ni-chelate acceptor beads for 1 hour at room temperature; followed by a 2-hour incubation with 15 nM biotin-PGC1α-3 peptide (QRRPCSELLKYLTTNDDPP) corresponding to amino acids 202 to 220.

The AlphaScreen signal was measured using an Envision Xcite plate reader (Perkin Elmer). Data was normalized, and curve fitting analysis was performed in GraphPad Prism 7 using a four-parameter dose-response fit.

All exemplified compounds of Formula I (Examples 1 - **86)** were found to have mean pIC₅₀ values above or equal to 5.

Examples **1, 2, 4, 5, 6, 7, 14, 17, 20, 21, 22, 24, 25,** 26, 49, 59, 60, 65, 66, 71, 72, 79, 81, 83 and 85 were found to have mean pIC₅₀ values above or equal to 6 but below 7.

Examples **10, 12, 15, 23, 28, 30, 31, 40, 41, 42, 43, 44, 45, 46, 47, 48, 52, 53, 54, 55, 56, 57, 58, 61, 64, 76, 77, 78** and **82** were found to have mean pIC₅₀ values above or equal to 7 but below 8.

Examples **27, 29, 32, 33, 34, 35, 36, 37, 38, 39, 63, 67, 68, 69, 70, 80, 84** and **86** were found to have mean pIC₅₀ values above or equal to 8.

### Example 88

### Full length ERRα reporter gene assay

Examples inhibitors **1, 2, 4, 5, 6, 7, 8, 9, 10, 11, 12, 13, 14, 15, 17, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 52, 53, 54, 55, 56, 57, 58, 59, 60, 61, 63, 64, 65, 66, 67, 68, 69, 70, 71, 72, 73, 74, 75, 76, 77, 78, 79, 80, 81, 82, 83, 84, 85** and **86** were tested for their ability to inhibit ERRα activity in a full length ERRα reporter gene assay.

A method was established to quantitatively screen the potency of compounds with inverse agonistic activity on the nuclear receptor ERRα of the human species. The assay allows intra-cellular screening of ERRα inverse agonists in SK-BR-3 cells using an over-expression construct coding full length ERRα and a reporter construct containing an ERRα Response Element (RE) and a luciferase gene for read out. The activity is expressed in loglC50 values and can be used to determine SAR of compound families or to de-select compounds.

In this assay, reporter cells are obtained by transient co-transfection of two constructs in SK-BR-3 cells using standard transfection techniques. The first construct contains a response element of the nuclear receptor ERRα (Plasmid pLP2175, Reporter construct ERRα-RE/Iuc2P, cloned variant of ERRa_v2_synthRE, Switchgear Genomics, Catalog Number S900089). This sequence drives the transcription of the luciferase reporter gene in response to binding of an ERRα protein encoded by the second construct (Plasmid pLP2124: full length ERRα expression construct using pcDNA3.1/Hygro(+) as background, Invitrogen Catalog Number V87020). The over-expressed full length ERRα is constitutively active, hence luciferase expression is reduced by inverse agonists of the nuclear receptor ERRα.

The day after transfection, cells were plated into 96 well plates, the test compound was added and the plates were incubated overnight. Subsequently, the firefly luciferase activity was quantified using luciferase detection reagent and luminescence readout.

### Detailed assay description

Transfection is performed on pre-seeded SK-BR-3 cells in a T175 flask. One transfected T175 flask is sufficient for seeding 3 to 4 MW96 plates the next day, depending on the confluency of the transfected cells.

Two different media are used in this protocol for cell treatment: 1) Culture medium, i.e. McCoy's 5a with phenol red (BioWhittaker Supplier Number 12-688F), 10% FBS and 1x Penstrep.; and, 2) Assay medium, i.e. McCoy's 5a Medium phenol red free (HyClone Product Code SH30270.01) with 2% Charcoal Stripped FBS and 1x Penstrep. Compound dilutions are prepared in assay medium.

Cells are seeded at least 2 days in advance to allow the cells to adhere well to flask before transfection. Cells should be 50-80% confluent at the day of transfection.

SKBR3 cells were transfected with the transcriptional reporter construct pLP2175 and the ERRα expression construct pLP2124 (as described above).

68 µL of Lipofectamine LTX transfection reagent (Invitrogen Catalog Number 15338-100) was added dropwise to 8.9 ml Opti-MEM I Reduced Serum Medium (Gibco Catalog Number 51985-026) and incubated at room temperature for 5 to 20 minutes. 8.9 ml of this reagent mixture was added to 22 µg pLP2175 +22 µg pLP2124 (ratio 1:1 and total volume 10 ml), and incubated at room temperature for 25 minutes.

10 minutes before adding the transfection mix to SKBR3 cells in a T175 flask, the culture medium was refreshed with 20 mL culture medium. Subsequently, the 10 mL DNA-Opti-MEM-Lipofectamine mixture was gently added to the cells, followed by overnight (16-24 hours) incubation at 37°C and 5% CO₂.

To harvest SKBR3 cells from a T175 flask, first the medium was removed. Subsequently, cells were washed with Phosphate Buffered Saline (PBS) (Lonza), after which the PBS was removed. To dissociate the cells, 2 ml of TrypLE Express (Invitrogen) was added to the flask, followed by incubation at 37°C for 5 minutes. The flask was tapped until cells were detached from the bottom. Cells were collected in 8 mL of culture medium (McCoy's 5a, 10% FBS, penstrep), to achieve a single cell suspension. After cell count, cells were spun down for 5 minutes at 300 g. Next cell pellets were re-suspended to 25000 cells/80 µl assay medium (McCoy's 5a phenol red free, 2% charcoal stripped FBS, penstrep).

For compound screening, the cells were harvested (as described above). 80 µL. of cell suspension (25,000 cells) was plated per well into a white, flat bottom, tissue culture treated, 96 well screening plates (Greiner).

Test compounds were diluted, starting from a 10 mM dimethylsulfoxide (DMSO) stock solution, in 3 dilution steps. The first dilution step was a 12 points serial dilution of 4-fold in DMSO. These dilutions were further diluted 10 times in phenol red free assay medium containing 2% charcoal stripped FBS and penstrep. The last step was another 20-fold dilution in assay medium to obtain a 5x concentrated dilution with a DMSO concentration of 0.5%. As a last step, the compound dilutions were diluted 5x in the cell plate.

The DMSO dilution series consisted of 12 concentrations, with a final concentration in the cell plate ranging from 10 µM to 2.4 fM.

The plates were incubated overnight (16-24 hours) at 37°C and 5% CO₂.

For the luciferase readout, the luciferase reagent (BriteLite Plus, Perkin Elmer) was brought to room temperature. To each test well of the screening plates, 100 µL of 2.5-fold diluted BriteLite Plus reagent was added, followed by incubation at room temperature for 5 minutes. The luciferase luminescence signal was measured using a Wallac Victor Microplate Reader (Perkin Elmer).

The half maximum inhibitory concentration (IC50) values for the test compounds were calculated from the luciferase signal using GraphPad Prism software (GraphPad Software).

From the exemplified compounds of Formula I, Examples **10, 29, 32, 33, 34, 35, 37, 38, 39, 40, 41, 43, 63, 67, 68, 69, 70, 84** and **86** were found to have mean plC50 values above or equal to 6.5.

### Example 89

### B16F10 Melanoma Syngeneic Mouse Model

Example inhibitor **80** was tested for its ability to inhibit tumor growth in a B16F10 melanoma syngeneic mouse model.

Cell line and tumor model: B16F10 melanoma cell line derived allograft model in C57BL/6 mice.

Mouse B16F10 melanoma cells were sourced from American Type Culture Collection (ATCC), USA. Cells were grown in DMEM (Invitrogen, Catalogue No. 31600-034) supplemented with 10% FBS (Invitrogen, Catalogue No. 10438-026), and 1% penicillin streptomycin (Thermo Fisher Scientific, Catalogue No. 15140-122). To establish allografts, the cells were harvested by trypsinization when they reach around 70 to 80 % confluence and 0.1 million B16F10 cells were suspended in 50 µl of serum free medium and mixed at 1:1 ratio with matrigel before implanting subcutaneously into the dorsal right flank of mice using a 1 mL BD syringe attached to a 24-gauge needle.

B16F10 tumor grafts were measured after 5 days of cell inoculation once they became palpable. When the average tumor volume reached around 58 mm³, animals were dosed after randomization into different treatment groups keeping tumor volume and number of animals in such a way so that the average tumor volume of each group remained same across the groups. Dosing was performed on a per kilogram weight basis, by mouth (*per os*, *p.o*.) once a day (quaque die, q.d).

Tumor dimensions - length (I) and breadth (b) - were measured by caliper on the day of animal randomization based on tumor volume (Day 1) and thrice weekly thereafter until study termination. Tumor volumes were calculated using the formula b² * l * 0.52 where l=length, b=breadth (Dusan Djokovic et al., BMC Cancer, 2010, 10:641). Tumor growth inhibition was calculated after normalizing the tumor volume on a given day to that on Day 1.

Test items administration was started when tumor volume reached an average of 58 mm³.

Administration of test items was carried out until Day 14 and measurement of tumor volume was carried out until Day 13 for computing percent tumor growth inhibition (TGI). The results of the study are listed in Table 1 herein below.

**Table 1**

| **Example** | **Dose** | **Tumor Volume (mm³)** | **Tumor Growth Inhibition (%)** |
|---|---|---|---|
| | **Milligrams per kilogram weight (mpk)** | **Day 13** | |
| | **[p.o., q.d]** | | |
| Vehicle Control | | 1801 ± 241 | - |
| 80 | 30 | 684 ± 82 | 64 |

In view of the foregoing description and examples, it will be apparent to those skilled in the art that equivalent modifications thereof can be made without departing from the scope of the claims.

## Claims

1. A compound according to Formula I 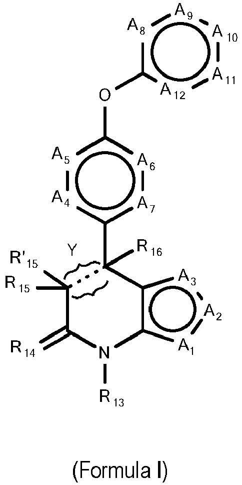 or a pharmaceutically acceptable salt thereof wherein
Y is a single carbon-carbon bond or a double carbon-carbon bond, with the proviso that when Y is a double carbon-carbon bond, R'₁₅ and R₁₆ are not present;
one of the three positions A₁-A₃ is either S or NR_{A}, the remaining two positions A₁-A₃ are N or CR₁, CR₂, CR₃, respectively;
R_{A} is H or methyl;
R₁-R₃ are independently H, methyl, amino or halogen;
A₄-A₇ are CR₄, CR₅, CR₆ and CR₇, respectively;
A₈-A₁₂ are N or CR₈, CR₉, CR₁₀, CR₁₁ and CR₁₂, respectively, with the proviso that no more than two of the five positions A₈ - A₁₂ can be simultaneously N;
R₄ - R₇ are independently H, halogen, C(1-3)alkoxy, C(1-3)alkyl;
R₈ - R₁₂ are independently H, halogen, C(1-3)alkoxy, C(1-4)alkyl, cyano, nitro, -C(=O)OR₁₇ , hydroxyl, C(3-6)cycloalkyl, benzyl, phenyl, -SF₅, bicyclo[1.1.1]pentane;
or R₉ and either R₈ or R₁₀ are fused and form an aromatic or non-aromatic five to seven membered ringcontaining two to seven carbon atoms and zero to three hetero atoms; with all carbon atoms optionally substituted with one or more methyl, halogen or hydroxyl;
R₁₃ is H or methyl;
R₁₄ is NH, O or S;
R'₁₅ is H, halogen, C(1-4)alkyl, -C(=O)OR₁₇ or -C(=O)NR₁₇R₁₇;
R₁₅ is H, halogen or C(1-4)alkyl;
R₁₆ is H; and,
R₁₇ is H, methyl or ethyl.

2. The compound according to claim 1, wherein A₁ is N, A₂ is NR_{A} and A₃ is CR₃, in particular wherein A₁ is N, A₂ is NH and A₃ is CH.

3. The compound according to claim 1 or 2, wherein R₅ is C(1-3)alkoxy and R₄, R₆ and R₇ are H, in particular wherein R₅ is methoxy and R₄, R₆ and R₇ are H.

4. The compound according to any one of claims 1 to 3, wherein A₈-A₁₂ are CR₈, CR₉, CR₁₀, CR₁₁ and CR₁₂ respectively, in particular wherein R₈-R₁₂ are independently H, C(1-4)alkyl, halogen, hydroxyl or C(1-3)alkoxy, more in particular wherein R₉ and R₁₁ are C(1-4)alkyl and R₈, R₁₀ and R₁₂ are H, more in particular wherein R₉ and R₁₁ are CF₃ and R₈, R₁₀ and R₁₂ are H.

5. The compound according to any one of claims 1 to 4, wherein R₁₃ is H; and/or wherein R₁₄ is O; and/or wherein Y is a single carbon-carbon bond.

6. The compound according to any one of claims 1 to 5, wherein R₁₅ and R'₁₅ are H.

7. The compound according to claim 1, which is selected from the group consisting of:
4-[3-methoxy-4-(3-methylphenoxy)phenyl]-*2H,4H,5H,6H,7H*-pyrazolo[3,4-*b*]pyridin-6-one;
4-(2-methoxy-4-{6-oxo-*2H,4H,5H,6H,7H*-pyrazolo[3,4-*b*]pyridin-4-yl}phenoxy)-3-(trifluoromethyl)benzonitrile;
4-[3-methoxy-4-(2-nitrophenoxy)phenyl]-*2H,4H,5H,6H,7H*-pyrazolo[3,4-*b*]pyridin-6-one;
4-{3-methoxy-4-[2-(trifluoromethyl)phenoxy]phenyl}-*2H,4H,5H,6H,7H*-pyrazolo[3,4-*b*]pyridin-6-one;
4-{3-methoxy-4-[4-(trifluoromethyl)phenoxy]phenyl}-*2H,4H,5H,6H,*7*H*-pyrazolo[3,4-*b*]pyridin-6-one;
4-{3-methoxy-4-[3-(trifluoromethyl)phenoxy]phenyl}-*2H,4H,5H,6H,*7*H*-pyrazolo[3,4-*b*]pyridin-6-one;
4-[4-(2-bromophenoxy)-3-methoxyphenyl]-*2H,4H,5H,6H,7H*-pyrazolo[3,4-*b*]pyridin-6-one;
4-[3-methoxy-4-(2-methylphenoxy)phenyl]-*2H,4H,5H,6H,7H*-pyrazolo[3,4-*b*]pyridin-6-one;
2-(2-methoxy-4-{6-oxo-*2H,4H,5H,6H,7H*-pyrazolo[3,4-*b*]pyridin-4-yl}phenoxy)benzonitrile;
4-[4-(2-*tert*-butylphenoxy)-3-methoxyphenyl]-*2H*,*4H*,*5H*,*6H*,*7H*-pyrazolo[3,4-*b*]pyridin-6-one;
4-[4-(2-chlorophenoxy)-3-methoxyphenyl]-*2H*,*4H*,*5H*,*6H*,*7H*-pyrazolo[3,4-*b*]pyridin-6-one;
4-{3-methoxy-4-[2-(trifluoromethoxy)phenoxy]phenyl}-*2H,4H,5H,6H,7H-*pyrazolo[3,4-*b*]pyridin-6-one;
4-[3-methoxy-4-(2-methoxyphenoxy)phenyl]-*2H,4H,5H,6H,7H*-pyrazolo[3,4-*b*]pyridin-6-one;
4-[4-(3-bromophenoxy)-3-methoxyphenyl]-*2H,4H,5H,6H,7H*-pyrazolo[3,4-*b*]pyridin-6-one;
4-[4-(3-*tert*-butylphenoxy)-3-methoxyphenyl]-*2H,4H,5H,6H,7H*-pyrazolo[3,4-*b*]pyridin-6-one;
4-[4-(3-chlorophenoxy)-3-methoxyphenyl]-*2H,4H,5H,6H,7H*-pyrazolo[3,4-*b*]pyridin-6-one;
4-{3-methoxy-4-[3-(trifluoromethoxy)phenoxy]phenyl}-*2H,4H,5H,6H,7H*-pyrazolo[3,4-*b*]pyridin-6-one;
4-[3-methoxy-4-(3-methoxyphenoxy)phenyl]-*2H*,*4H*,*5H*,*6H*,*7H*-pyrazolo[3,4-*b*]pyridin-6-one;
methyl 3-(2-methoxy-4-{6-oxo-*2H,4H,5H,6H,7H*-pyrazolo[3,4-*b*]pyridin-4-yl}phenoxy)benzoate;
4-[4-(2-ethylphenoxy)-3-methoxyphenyl]-*2H,4H,5H,6H,7H*-pyrazolo[3,4-*b*]pyridin-6-one;
4-[4-(3-ethylphenoxy)-3-methoxyphenyl]-*2H,4H,5H,6H,7H*-pyrazolo[3,4-*b*]pyridin-6-one;
4-[3-methoxy-4-(2-propylphenoxy)phenyl]-*2H,4H,5H,6H,7H*-pyrazolo[3,4-*b*]pyridin-6-one;
4-{3-methoxy-4-[2-(propan-2-yl)phenoxy]phenyl}-*2H,4H,5H,6H,7H*-pyrazolo[3,4-*b*]pyridin-6-one;
4-[3-methoxy-4-(2-phenylphenoxy)phenyl]-*2H,4H,5H,6H,7H*-pyrazolo[3,4-*b*]pyridin-6-one;
4-[4-(2-cyclopentylphenoxy)-3-methoxyphenyl]-*2H,4H,5H,6H,7H*-pyrazolo[3,4-*b*]pyridin-6-one;
4-[4-(2-benzylphenoxy)-3-methoxyphenyl]-*2H,4H,5H,6H,7H*-pyrazolo[3,4-*b*]pyridin-6-one;
4-[4-(2*-tert*-butyl-4-methoxyphenoxy)-3-methoxyphenyl]-*2H,4H,5H,6H,7H*-pyrazolo[3,4-*b*]pyridin-6-one;
4-[4-(2-cyclopropylphenoxy)-3-methoxyphenyl]-*2H,4H,5H,6H,7H*-pyrazolo[3,4-*b*]pyridin-6-one;
4-[4-(3-*tert*-butyl-4-hydroxyphenoxy)-3-methoxyphenyl]-*2H,4H,5H,6H,7H*-*pyrazolo*[3,4-*b*]pyridin-6-one;
4-{3-methoxy-4-[3-(propan-2-yl)phenoxy]phenyl}-*2H,4H,5H,6H,7H*-pyrazolo[3,4-*b*]pyridin-6-one;
4-[4-(3-cyclopropylphenoxy)-3-methoxyphenyl]-*2H,4H,5H,6H,7H*-pyrazolo[3,4-*b*]pyridin-6-one;
4-[4-(2-*tert*-butyl-4-ethylphenoxy)-3-methoxyphenyl]-*2H,4H,5H,6H,7H-*pyrazolo[3,4-*b*]pyridin-6-one;
4-[4-(4-bromo-2-*tert*-butylphenoxy)-3-methoxyphenyl]-*2H,4H,5H,6H,7H*-pyrazolo[3,4-*b*]pyridin-6-one;
4-[4-(2*-tert*-butyl-4-methylphenoxy)*-*3-methoxyphenyl]*-2H,4H,5H,6H,7H*-pyrazolo[3,4-*b*]pyridin-6-one;
methyl 3-*tert*-butyl-4-(2-methoxy-4-{6-oxo-*2H,4H,5H,6H,7H*-pyrazolo[3,4-*b*]pyridin-4-yl}phenoxy)benzoate;
methyl 4-(2-methoxy-4-{6-oxo-*2H,4H,5H,6H,7H*-pyrazolo[3,4-*b*]pyridin-4-yl}phenoxy)naphthalene-1-carboxylate;
4-[4-(2,4-di-*tert*-butylphenoxy)-3-methoxyphenyl]-*2H,4H,5H,6H,7H*-pyrazolo[3,4-*b*]pyridin-6-one;
4-{3-methoxy-4-[3-methoxy-5-(trifluoromethyl)phenoxy]phenyl}-*2H,4H,5H,6H,7H*-pyrazolo[3,4-*b*]pyridin-6-one;
3-*tert*-butyl-4-(2-methoxy-4-{6-oxo-*2H,4H,5H,6H,7H-*pyrazolo[3,4-*b*]pyridin-4-yl}phenoxy)benzonitrile;
4-{3-methoxy-4-[2-(pentafluoro-λ⁶-sulfanyl)phenoxy]phenyl}-*2H,4H,5H,6H,7H*-pyrazolo[3,4-*b*]pyridin-6-one;
methyl 3-(2-methoxy-4-{6-oxo-*2H,4H,5H,6H,7H*-pyrazolo[3,4-*b*]pyridin-4-yl}phenoxy)-5-(trifluoromethyl)benzoate;
4-{4-[3-bromo-5-(trifluoromethyl)phenoxy]-3-methoxyphenyl}-*2H,4H,5H,6H,7H*-pyrazolo[3,4-*b*]pyridin-6-one;
4-{4-[3,5-bis(trifluoromethyl)phenoxy]-3-methoxyphenyl}-*2H,4H,5H,6H,7H-*pyrazolo[3,4-*b*]pyridin-6-one;
4-[4-(3,5-di-*tert*-butylphenoxy)-3-methoxyphenyl]-*2H,4H,5H,6H,7H*-pyrazolo[3,4-*b*]pyridin-6-one;
4-[4-(2-{bicyclo[1.1.1]pentan-1-yl}phenoxy)-3-methoxyphenyl]-*2H,4H,5H,6H,7H*-pyrazolo[3,4-*b*]pyridin-6-one;
4-(2-methoxy-4-{6-oxo-*2H,4H,5H,6H*,*7H*-pyrazolo[3,4-*b*]pyridin-4-yl}phenoxy)naphthalene-1-carbonitrile;
4-[4-(2,3-dihydro-*1H*-inden-4-yloxy)-3-methoxyphenyl]-*2H,4H,5H,6H,7H*-pyrazolo[3,4-*b*]pyridin-6-one;
4-{4-[(2,2-dimethyl-2,3-dihydro-1-benzofuran-7-yl)oxy]-3-methoxyphenyl}-*2H,4H,5H,6H*,*7H*-pyrazolo[3,4-*b*]pyridin-6-one;
4-[4-(2,3-dihydro-*1H*-inden-5-yloxy)-3-methoxyphenyl]-*2H,4H,5H,6H,7H*-pyrazolo[3,4-*b*]pyridin-6-one;
methyl 8-(2-methoxy-4-{6-oxo-*2H,4H,5H,6H,7H*-pyrazolo[3,4-*b*]pyridin-4-yl}phenoxy)quinoline-5-carboxylate;
4-(3-methoxy-4-phenoxyphenyl)-*2H,4H,5H,6H,7H-*pyrazolo[3,4-*b*]pyridin-6-one;
4-{3-bromo-4-[3-methoxy-5-(trifluoromethyl)phenoxy]phenyl}-*2H,4H,5H,6H,7H-*pyrazolo[3,4-*b*]pyridin-6-one;
3-*tert*-butyl-4-(4-{6-oxo-*2H,4H,5H,6H,7H*-pyrazolo[3,4-*b*]pyridin-4-yl}phenoxy)benzonitrile;
4-{4-[3-methoxy-5-(trifluoromethyl)phenoxy]phenyl}-*2H,4H,5H,6H,7H*-pyrazolo[3,4-*b*]pyridin-6-one;
3-*tert*-butyl-4-(2-methyl-4-{6-oxo-*2H,4H,5H,6H,7H*-pyrazolo[3,4-*b*]pyridin-4-yl}phenoxy)benzonitrile;
4-{4-[3-methoxy-5-(trifluoromethyl)phenoxy]-3-methylphenyl}-*2H,4H,5H,6H,7H*-pyrazolo[3,4-*b*]pyridin-6-one;
4-{3-fluoro-4-[3-methoxy-5-(trifluoromethyl)phenoxy]phenyl}-*2H,4H,5H,6H,7H*-pyrazolo[3,4-*b*]pyridin-6-one;
4-{4-[2-(pentafluoro-λ⁶-sulfanyl)phenoxy]-3-(trifluoromethoxy)phenyl}-*2H,4H,5H,6H,7H*-pyrazolo[3,4-*b*]pyridin-6-one;
4-{4-[2-(pentafluoro-λ⁶-sulfanyl)phenoxy]phenyl}-*2H*,*4H*,*5H*,*6H*,*7H*-pyrazolo[3,4-*b*]pyridin-6-one;
4-[3-(trifluoromethoxy)-4-[2-(trifluoromethyl)phenoxy]phenyl]-*2H,4H,5H,6H,7H*-pyrazolo[3,4-*b*]pyridin-6-one;
3-*tert*-butyl-4-(2-methoxy-4-{6-oxo-*2H,4H,5H,6H,7H*-pyrazolo[3,4-*b*]pyridin-4-yl}phenoxy)benzoic acid;
3-(2-methoxy-4-{6-oxo-*2H,4H,5H,6H,7H*-pyrazolo[3,4-*b*]pyridin-4-yl}phenoxy)-5-(trifluoromethyl)benzoic acid;
4-{3-methoxy-4-[3-methyl-5-(trifluoromethyl)phenoxy]phenyl}-*2H,4H,5H,6H,7H*-pyrazolo[3,4-*b*]pyridin-6-one;
4-[4-(3,5-dimethylphenoxy)-3-methoxyphenyl]-*2H,4H,5H,6H,7H*-pyrazolo[3,4-*b*]pyridin-6-one;
4-{3-methoxy-4-[2-methyl-4-(trifluoromethyl)phenoxy]phenyl}-*2H,4H,5H,6H,7H*-pyrazolo[3,4-*b*]pyridin-6-one;
4-[4-(2,4-dimethylphenoxy)-3-methoxyphenyl]-*2H,4H*,*5H,6H,7H*-pyrazolo[3,4-*b*]pyridin-6-one;
4-[4-(2-*tert*-butyl-4-ethylphenoxy)-3-methoxyphenyl]-*2H,6H,7H*-pyrazolo[3,4-*b*]pyridin-6-one;
4-[4-(2-*tert*-butyl-4-methoxyphenoxy)-3-methoxyphenyl]-*2H,6H,7H*-pyrazolo[3,4-*b*]pyridin-6-one;
4-[4-(2-*tert*-butyl-4-methylphenoxy)-3-methoxyphenyl]-*2H,6H,7H*-pyrazolo[3,4-*b*]pyridin-6-one;
4-[4-(4-bromo-2-*tert*-butylphenoxy)-3-methoxyphenyl]-*2H,6H,7H*-pyrazolo[3,4-*b*]pyridin-6-one;
4-(2-methoxy-4-{6-oxo-*2H,6H,7H*-pyrazolo[3,4-*b*]pyridin-4-yl}phenoxy)-3-(trifluoromethyl)benzonitrile;
methyl 4-{4-[3,5-bis(trifluoromethyl)phenoxy]-3-methoxyphenyl}-6-oxo-*2H,4H,5H,6H,7H*-pyrazolo[3,4-*b*]pyridine-5-carboxylate;
ethyl 4-{4-[3,5-bis(trifluoromethyl)phenoxy]-3-methoxyphenyl}-6-oxo-*2H,4H,5H,6H,7H*-pyrazolo[3,4-*b*]pyridine-5-carboxylate;
4-{4-[3,5-bis(trifluoromethyl)phenoxy]-3-methoxyphenyl}-*N*,*N*-dimethyl-6-oxo-*2H,4H,5H,6H,7H-*pyrazolo[3,4-*b*]pyridine-5-carboxamide;
4-{4-[3,5-bis(trifluoromethyl)phenoxy]-3-methoxyphenyl}-*N,N-*diethyl-6-oxo-*2H,4H,5H,6H,7H-*pyrazolo[3,4-*b*]pyridine-5-carboxamide;
4-{4-[3,5-bis(trifluoromethyl)phenoxy]-3-methoxyphenyl}-*1H,2H,3H,4H,6H*-pyrrolo[3,4-*b*]pyridin-2-one;
4-{4-[3-(difluoromethyl)-5-(trifluoromethyl)phenoxy]-3-methoxyphenyl}-*2H,4H,5H,6H,7H-*pyrazolo[3,4-*b*]pyridin-6-one;
4-{4-[2-(difluoromethyl)-4-(trifluoromethyl)phenoxy]-3-methoxyphenyl}-*2H,4H,5H,6H,7H-*pyrazolo[3,4-*b*]pyridin-6-one;
(+)-4-{4-[3,5-bis(trifluoromethyl)phenoxy]-3-methoxyphenyl}-*2H*,*4H*,*5H,6H*,*7H-*pyrazolo[3,4-*b*]pyridin-6-one;
(-)-4-{4-[3,5-bis(trifluoromethyl)phenoxy]-3-methoxyphenyl}-*2H,4H,5H,6H,7H*-pyrazolo[3,4-*b*]pyridin-6-one;
(+)-4-[4-(3,5-dimethylphenoxy)-3-methoxyphenyl]-*2H,4H,5H,6H,7H*-pyrazolo[3,4-*b*]pyridin-6-one;
(-)-4-[4-(3,5-dimethylphenoxy)-3-methoxyphenyl]-*2H,4H,5H,6H,7H*-pyrazolo[3,4-*b*]pyridin-6-one;
(+)-4-{3-methoxy-4-[3-methyl-5-(trifluoromethyl)phenoxy]phenyl}-*2H,4H,5H,6H,7H-*pyrazolo[3,4-*b*]pyridin-6-one;
(-)-4-{3-methoxy-4-[3-methyl-5-(trifluoromethyl)phenoxy]phenyl}-*2H,4H,5H,6H,7H*-pyrazolo[3,4-*b*]pyridin-6-one;
(+)-4-{4-[3-(difluoromethyl)-5-(trifluoromethyl)phenoxy]-3-methoxyphenyl}-*2H,4H,5H,6H*,*7H-*pyrazolo[3,4-*b*]pyridin-6-one; and,
(-)-4-{4-[3-(difluoromethyl)-5-(trifluoromethyl)phenoxy]-3-methoxyphenyl}-*2H,4H,5H,6H,7H-*pyrazolo[3,4-*b*]pyridin-6-one.

8. The compound according to claim 1, which is
(+)-4-{4-[3,5-bis(trifluoromethyl)phenoxy]-3-methoxyphenyl}-*2H,4H,5H,6H,7H*-pyrazolo[3,4-*b*]pyridin-6-one; or,
(-)-4-{4-[3,5-bis(trifluoromethyl)phenoxy]-3-methoxyphenyl}-*2H,4H,5H,6H,7H*-pyrazolo[3,4-*b*]pyridin-6-one.

9. Medicament **characterized in that** it comprises a compound of Formula I according to any one of claims 1 to 8 or a pharmaceutically acceptable salt thereof.

10. Compound according to any one of claims 1 to 8 or a pharmaceutically acceptable salt thereof for use in therapy.

11. Compound according to any one of claims 1 to 8 or a pharmaceutically acceptable salt thereof for use in the treatment of ERRα-mediated cancer.

12. Compound according to any one of claims 1 to 8 or a pharmaceutically acceptable salt thereof for use in the treatment of at least one condition selected from: lung cancer; melanoma; endometrial cancer; and, acute myeloid leukemia.

13. Compound according to any one of claims 1 to 8 or a pharmaceutically acceptable salt thereof for use in the treatment of at least one condition selected from: breast cancer; bladder cancer; prostate cancer; pancreatic cancer; colorectal cancer; and, ovarian cancer.

14. Compound according to any one of claims 1 to 8 or a pharmaceutically acceptable salt thereof for use in the treatment of Type II diabetes mellitus.

15. Pharmaceutical composition which comprises a compound of Formula I according to any one of claims 1 to 8 or a pharmaceutically acceptable salt thereof and one or more pharmaceutically acceptable excipients, in particular which pharmaceutical composition further comprises at least one additional therapeutically active agent.

## Patentansprüche

1. Eine Verbindung gemäß Formel I 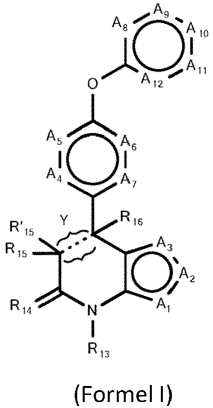 oder ein pharmazeutisch verträgliches Salz davon, wobei
Y eine Kohlenstoff-Kohlenstoff-Einfachbindung oder eine Kohlenstoff-Kohlenstoff-Doppelbindung ist, mit der Maßgabe, dass, wenn Y eine Kohlenstoff-Kohlenstoff-Doppelbindung ist, R'₁₅ und R₁₆ nicht vorhanden sind;
eine der drei Positionen A₁-A₃ entweder S oder NR_{A} ist, die verbleibenden zwei Positionen A₁-A₃ für N oder CR₁, CR₂ bzw. CR₃ stehen;
R_{A} für H oder Methyl steht;
R₁-R₃ unabhängig H, Methyl, Amino oder Halogen sind;
A₄-A₇ für CR₄, CR₅, CR₆ bzw. CR₇ stehen;
A₈ - A₁₂ für N oder CR₈, CR₉, CR₁₀, CR₁₁ bzw. CR₁₂ stehen, mit der Maßgabe, dass nicht mehr als zwei der fünf Positionen A₈ - A₁₂ gleichzeitig N sein können;
R₄ - R₇ unabhängig H, Halogen, C(1-3)-Alkoxy, C(1-3)-Alkyl sind;
R₈ - R₁₂ unabhängig H, Halogen, C(1-3)-Alkoxy, C(1-4)-Alkyl, Cyano, Nitro, -C(=O)OR₁₇, Hydroxyl, C(3-6)-Cycloalkyl, Benzyl, Phenyl, -SF₅, Bicyclo[1.1.1]pentan sind;
oder R₉ und entweder R₈ oder R₁₀ kondensiert sind und einen aromatischen oder nichtaromatischen fünf- bis siebengliedrigen Ring bilden, der zwei bis sieben Kohlenstoffatome und null bis drei Heteroatome enthält; wobei alle Kohlenstoffatome gegebenenfalls mit einem oder mehreren Methyl, Halogen oder Hydroxyl substituiert sind;
R₁₃ für H oder Methyl steht;
R₁₄ für NH, O oder S steht;
R'₁₅ für H, Halogen, C(1-4)-Alkyl, -C(=O)OR₁₇ oder -C(=O)NR₁₇R₁₇ steht;
R₁₅ für H, Halogen oder C(1-4)-Alkyl steht;
R₁₆ für H steht; und
R₁₇ für H, Methyl oder Ethyl steht.

2. Die Verbindung nach Anspruch 1, worin A₁ für N steht, A₂ für NR_{A} steht und A₃ für CR₃ steht, insbesondere wobei A₁ für N steht, A₂ für NH steht und A₃ für CH steht.

3. Die Verbindung nach Anspruch 1 oder 2, wobei R₅ für C(1-3)-Alkoxy steht und R₄, R₆ und R₇ für H stehen, insbesondere wobei R₅ Methoxy ist und R₄, R₆ und R₇ für H stehen.

4. Die Verbindung nach einem der Ansprüche 1 bis 3, wobei A₈-A₁₂ für CR₈, CR₉, CR₁₀, CR₁₁ bzw. CR₁₂ stehen, insbesondere wobei R₈-R₁₂ unabhängig H, C(1-4)-Alkyl, Halogen, Hydroxyl oder C(1-3)-Alkoxy sind, noch mehr insbesondere wobei R₉ und R₁₁ für C(1-4)-Alkyl stehen und R₈, R₁₀ und R₁₂ für H stehen, noch mehr insbesondere wobei R₉ und R₁₁ für CF₃ stehen und R₈, R₁₀ und R₁₂ für H stehen.

5. Die Verbindung nach einem der Ansprüche 1 bis 4, wobei R₁₃ für H steht; und/oder wobei R₁₄ für O steht; und/oder wobei Y eine Kohlenstoff-Kohlenstoff-Einfachbindung ist.

6. Die Verbindung nach einem der Ansprüche 1 bis 5, wobei R₁₅ und R'₁₅ für H stehen.

7. Die Verbindung nach Anspruch 1, die ausgewählt ist aus der Gruppe bestehend aus:
4-[3-Methoxy-4-(3-methylphenoxy)phenyl]-*2H,4H,5H,6H,7H*-pyrazolo[3,4-*b*]pyridin-6-on;
4-(2-Methoxy-4-{6-oxo-*2H,4H,5H,6H,7H*-pyrazolo[3,4-*b*]pyridin-4-yl}phenoxy)-3-(trifluormethyl)benzonitril;
4-[3-Methoxy-4-(2-nitrophenoxy)phenyl]-*2H,4H,5H,6H,7H*-pyrazolo[3,4-*b*]pyridin-6-on;
4-{3-Methoxy-4-[2-(trifluormethyl)phenoxy]phenyl}-*2H,4H,5H,6H,7H*-pyrazolo[3,4-*b*]pyridin-6-on;
4-{3-Methoxy-4-[4-(trifluormethyl)phenoxy]phenyl}-*2H,4H,5H,6H,7H*-pyrazolo[3,4-*b*]pyridin-6-on;
4-{3-Methoxy-4-[3-(trifluormethyl)phenoxy]phenyl}-*2H,4H,5H,6H,7H*-pyrazolo[3,4-*b*]pyridin-6-on;
4-[4-(2-Bromphenoxy)-3-methoxyphenyl]-*2H,4H,5H,6H,7H*-pyrazolo[3,4-*b*]pyridin-6-on;
4-[3-Methoxy-4-(2-methylphenoxy)phenyl]-*2H,4H,5H,6H,7H*-pyrazolo[3,4-*b*]pyridin-6-on;
2-(2-Methoxy-4-{6-oxo-*2H,4H,5H,6H,7H*-pyrazolo[3,4-*b*]pyridin-4-yl}phenoxy)benzonitril;
4-[4-(2-*tert*-Butylphenoxy)-3-methoxyphenyl]-*2H,4H,5H,6H,7H*-pyrazolo[3,4-*b*]pyridin-6-on;
4-[4-(2-Chlorphenoxy)-3-methoxyphenyl]-*2H,4H,5H,6H,7H*-pyrazolo[3,4-*b*]pyridin-6-on;
4-{3-Methoxy-4-[2-(trifluormethoxy)phenoxy]phenyl}-*2H,4H,SH,6H,7H*-pyrazolo[3,4-*b*]pyridin-6-on;
4-[3-Methoxy-4-(2-methoxyphenoxy)phenyl]-*2H,4H,5H,6H,7H*-pyrazolo[3,4-*b*]pyridin-6-on;
4-[4-(3-Bromphenoxy)-3-methoxyphenyl]-*2H,4H,5H,6H,7H*-pyrazolo[3,4-*b*]pyridin-6-on;
4-[4-(3-*tert*-Butylphenoxy)-3-methoxyphenyl]-*2H,4H,5H,6H,7H*-pyrazolo[3,4-*b*]pyridin-6-on;
4-[4-(3-Chlorphenoxy)-3-methoxyphenyl]-*2H,4H,5H,6H,7H*-pyrazolo[3,4-*b*]pyridin-6-on;
4-{3-Methoxy-4-[3-(trifluormethoxy)phenoxy]phenyl}-*2H,4H,5H,6H,7H*-pyrazolo[3,4-*b*]pyridin-6-on;
4-[3-Methoxy-4-(3-methoxyphenoxy)phenyl]-*2H,4H,5H,6H,7H*-pyrazolo[3,4-*b*]pyridin-6-on;
Methyl-3-(2-methoxy-4-{6-oxo-*2H,4H,5H,6H,7H*-pyrazolo[3,4-*b*]pyridin-4-yl}phenoxy)benzoat;
4-[4-(2-Ethylphenoxy)-3-methoxyphenyl]-*2H,4H,5H,6H,7H*-pyrazolo[3,4-*b*]pyridin-6-on;
4-[4-(3-Ethylphenoxy)-3-methoxyphenyl]-*2H,4H,5H,6H,7H*-pyrazolo[3,4-*b*]pyridin-6-on;
4-[3-Methoxy-4-(2-propylphenoxy)phenyl]-*2H,4H,5H,6H*,*7H*-pyrazolo[3,4-*b*]pyridin-6-on;
4-{3-Methoxy-4-[2-(propan-2-yl)phenoxy]phenyl}-*2H,4H,5H,6H,7H*-pyrazolo[3,4-*b*]pyridin-6-on;
4-[3-Methoxy-4-(2-phenylphenoxy)phenyl]-*2H,4H,5H,6H,7H-*pyrazolo[3,4-*b*]pyridin-6-on;
4-[4-(2-Cyclopentylphenoxy)-3-methoxyphenyl]-*2H*,*4H*,*5H*,*6H*,*7H*-pyrazolo[3,4-*b*]pyridin-6-on;
4-[4-(2-Benzylphenoxy)-3-methoxyphenyl]-*2H,4H,5H,6H,7H*-pyrazolo[3,4-*b*]pyridin-6-on;
4-[4-(2-*tert*-Butyl-4-methoxyphenoxy)-3-methoxyphenyl]-*2H,4H,5H,6H,7H*-pyrazolo[3,4-*b*]pyridin-6-on;
4-[4-(2-Cyclopropylphenoxy)-3-methoxyphenyl]-*2H,4H,5H,6H,7H*-pyrazolo[3,4-*b*]pyridin-6-on;
4-[4-(3-*tert*-Butyl-4-hydroxyphenoxy)-3-methoxyphenyl]-*2H,4H,5H,6H,7H*-pyrazolo[3,4-*b*]pyridin-6-on;
4-{3-Methoxy-4-[3-(propan-2-yl)phenoxy]phenyl}-*2H,4H,5H,6H,7H*-pyrazolo[3,4-*b*]pyridin-6-on;
4-[4-(3-Cyclopropylphenoxy)-3-methoxyphenyl]-*2H,4H,5H,6H,7H*-pyrazolo[3,4-*b*]pyridin-6-on;
4-[4-(2-*tert*-Butyl-4-ethylphenoxy)-3-methoxyphenyl]-*2H,4H,5H,6H,7H*-pyrazolo[3,4-*b*]pyridin-6-on;
4-[4-(4-Brom-2-*tert*-butylphenoxy)-3-methoxyphenyl]-*2H,4H,5H,6H,7H*-pyrazolo[3,4-*b*]pyridin-6-on;
4-[4-(2-*tert*-Butyl-4-methylphenoxy)-3-methoxyphenyl]-*2H,4H,5H,6H,7H*-pyrazolo[3,4-*b*]pyridin-6-on;
Methyl-3-*tert*-butyl-4-(2-methoxy-4-{6-oxo-*2H*,*4H*,*5H*,*6H*,*7H*-pyrazolo[3,4-*b*]pyridin-4-yl}phenoxy)benzoat;
Methyl-4-(2-methoxy-4-{6-oxo-*2H,4H,5H,6H,7H*-pyrazolo[3,4-*b*]pyridin-4-yl}phenoxy)naphthalin-1-carboxylat;
4-[4-(2,4-Di-*tert*-butylphenoxy)-3-methoxyphenyl]-*2H,4H,5H,6H,7H*-pyrazolo[3,4-*b*]pyridin-6-on;
4-{3-Methoxy-4-[3-methoxy-5-(trifluormethyl)phenoxy]phenyl}-*2H,4H,5H,6H,7H-*pyrazolo[3,4-*b*]pyridin-6-on;
3-*tert*-Butyl-4-(2-methoxy-4-{6-oxo-*2H*,*4H*,*5H*,*6H*,*7H*-pyrazolo[3,4-*b*]pyridin-4-yl}phenoxy)benzonitril;
4-{3-methoxy-4-[2-(pentafluor-λ⁶-sulfanyl)phenoxy]phenyl}-*2H,4H,5H,6H,7H-*pyrazolo[3,4-*b*]pyridin-6-on;
Methyl-3-(2-Methoxy-4-{6-oxo-*2H,4H,5H,6H,7H*-pyrazolo[3,4-*b*]pyridin-4-yl}phenoxy)-5-(trifluormethyl)benzoat;
4-{4-[3-Brom-5-(trifluormethyl)phenoxy]-3-methoxyphenyl}-*2H,4H,5H,6H,7H-*pyrazolo[3,4-*b*]pyridin-6-on;
4-{4-[3,5-Bis(trifluormethyl)phenoxy]-3-methoxyphenyl}-*2H*,*4H*,*5H*,*6H*,*7H*-pyrazolo[3,4-*b*]pyridin-6-on;
4-[4-(3,5-Di-tert-butylphenoxy)-3-methoxyphenyl]-*2H,4H,5H,6H,7H*-pyrazolo[3,4-*b*]pyridin-6-on;
4-[4-(2-{Bicyclo[1.1.1]pentan-1-yl}phenoxy)-3-methoxyphenyl]-*2H,4H,5H,6H,7H-*pyrazolo[3,4-*b*]pyridin-6-on;
4-(2-Methoxy-4-{6-oxo-*2H,4H,5H*,*6H,7H-*pyrazolo[3,4-*b*]pyridin-4-yl}phenoxy)naphthalin-1-carbonitril;
4-[4-(2,3-Dihydro-1H-inden-4-yloxy)-3-methoxyphenyl]-*2H,4H,5H,6H,7H*-pyrazolo[3,4-*b*]pyridin-6-on;
4-{4-[(2,2-Dimethyl-2,3-dihydro-1-benzofuran-7-yl)oxy]-3-methoxyphenyl}-*2H,4H,5H,6H,7H*-pyrazolo[3,4-*b*]pyridin-6-on;
4-[4-(2,3-Dihydro-1H-inden-5-yloxy)-3-methoxyphenyl]-*2H,4H,5H,6H,7H*-pyrazolo[3,4-*b*]pyridin-6-on;
Methyl-8-(2-methoxy-4-{6-oxo-*2H,4H,5H,6H,7H*-pyrazolo[3,4-*b*]pyridin-4-yl}phenoxy)chinolin-5-carboxylat;
4-(3-Methoxy-4-phenoxyphenyl)-*2H,4H,5H,6H,7H*-pyrazolo[3,4-*b*]pyridin-6-on;
4-{3-*Brom*-4-[3-methoxy-5-(trifluormethyl)phenoxy]phenyl}-*2H,4H,5H,6H,7H-*pyrazolo[3,4-*b*]pyridin-6-on;
3-*tert*-Butyl-4-(4-{6-oxo-*2H*,*4H,5H,6H,7H*-pyrazolo[3,4-*b*]pyridin-4-yl}phenoxy)benzonitril;
4-{4-[3-Methoxy-5-(trifluormethyl)phenoxy]phenyl}-*2H,4H,5H,6H,7H*-pyrazolo[3,4-*b*]pyridin-6-on;
3-*tert*-Butyl-4-(2-methyl-4-{6-oxo-*2H,4H,5H,6H,7H*-pyrazolo[3,4-*b*]pyridin-4-yl}phenoxy)benzonitril;
4-{4-[3-Methoxy-5-(trifluormethyl)phenoxy]-3-methylphenyl}-*2H,4H,5H,6H,7H-*pyrazolo[3,4-*b*]pyridin-6-on;
4-{3-Fluor-4-[3-methoxy-5-(trifluormethyl)phenoxy]phenyl}-*2H,4H,5H,6H,7H-*pyrazolo[3,4-*b*]pyridin-6-on;
4-{4-[2-(Pentafluor-λ⁶-sulfanyl)phenoxy]-3-(trifluormethoxy)phenyl}-*2H,4H,5H,6H,7H-*pyrazolo[3,4-*b*]pyridin-6-on;
4-{4-[2-(Pentafluor-λ⁶-sulfanyl)phenoxy]phenyl}-*2H,4H,5H,6H,7H*-pyrazolo[3,4-*b*]pyridin-6-on;
4-[3-(Trifluormethoxy)-4-[2-(trifluormethyl)phenoxy]phenyl]-*2H,4H,5H,6H,7H-*pyrazolo[3,4-*b*]pyridin-6-on;
3-*tert*-Butyl-4-(2-methoxy-4-{6-oxo-*2H,4H,5H,6H,7H*-pyrazolo[3,4-*b*]pyridin-4-yl}phenoxy)benzoesäure;
3-(2-Methoxy-4-{6-oxo-*2H,4H,5H,6H,7H*-pyrazolo[3,4-*b*]pyridin-4-yl}phenoxy)-5-(trifluormethyl)benzoesäure;
4-{3-Methoxy-4-[3-methyl-5-(trifluormethyl)phenoxy]phenyl}-*2H,4H,5H,6H,7H-*pyrazolo[3,4-*b*]pyridin-6-on;
4-[4-(3,5-Dimethylphenoxy)-3-methoxyphenyl]-*2H,4H,5H,6H,7H*-pyrazolo[3,4-*b*]pyridin-6-on;
4-{3-Methoxy-4-[2-methyl-4-(trifluormethyl)phenoxy]phenyl}-*2H,4H,5H,6H,7H-*pyrazolo[3,4-*b*]pyridin-6-on;
4-[4-(2,4-Dimethylphenoxy)-3-methoxyphenyl]-*2H,4H,5H,6H,7H-*pyrazolo[3,4-*b*]pyridin-6-on;
4-[4-(2-*tert*-Butyl-4-ethylphenoxy)-3-methoxyphenyl]-*2H,6H,7H*-pyrazolo[3,4-*b*]pyridin-6-on;
4-[4-(2-*tert*-Butyl-4-methoxyphenoxy)-3-methoxyphenyl]-*2H,6H,7H*-pyrazolo[3,4-*b*]pyridin-6-on;
4-[4-(2-*tert*-Butyl-4-methylphenoxy)-3-methoxyphenyl]-*2H,6H,7H*-pyrazolo[3,4-*b*]pyridin-6-on;
4-[4-(4-Brom-2-*tert*-butylphenoxy)-3-methoxyphenyl]-*2H,6H,7H*-pyrazolo[3,4-*b*]pyridin-6-on;
4-(2-Methoxy-4-{6-oxo-2*H*,*6H,7H*-pyrazolo[3,4-*b*]pyridin-4-yl}phenoxy)-3-(trifluormethyl)benzonitril;
Methyl-4-{4-[3,5-bis(trifluormethyl)phenoxy]-3-methoxyphenyl}-6-oxo-*2H,4H,5H,6H,7H-*pyrazolo[3,4-*b*]pyridin-5-carboxylat;
Ethyl-4-{4-[3,5-bis(trifluormethyl)phenoxy]-3-methoxyphenyl}-6-oxo-*2H,4H,5H,6H,7H-*pyrazolo[3,4-*b*]pyridin-5-carboxylat;
4-{4-[3,5-Bis(trifluormethyl)phenoxy]-3-methoxyphenyl}-*N,N*-dimethyl-6-oxo-*2H,4H,5H,6H,*7*H*-pyrazolo[3,4-*b*]pyridin-5-carboxamid;
4-{4-[3,5-Bis(trifluormethyl)phenoxy]-3-methoxyphenyl}-*N,N-*diethyl-6-oxo-*2H,4H,5H,6H,7H*-pyrazolo[3,4-*b*]pyridin-5-carboxamid;
4-{4-[3,5-Bis(trifluormethyl)phenoxy]-3-methoxyphenyl}-*1H,2H,3H,4H,6H*-pyrrolo[3,4-*b*]pyridin-2-on;
4-{4-[3-(Difluormethyl)-5-(trifluormethyl)phenoxy]-3-methoxyphenyl}-*2H,4H,5H,6H,7H-*pyrazolo[3,4-*b*]pyridin-6-on;
4-{4-[2-(Difluormethyl)-4-(trifluormethyl)phenoxy]-3-methoxyphenyl}-*2H,4H,5H,6H,7H-*pyrazolo[3,4-*b*]pyridin-6-on;
(+)-4-{4-[3,5-Bis(trifluormethyl)phenoxy]-3-methoxyphenyl}-*2H,4H,5H,6H,7H-*pyrazolo[3,4-*b*]pyridin-6-on;
(-)-4-{4-[3,5-Bis(trifluormethyl)phenoxy]-3-methoxyphenyl}-*2H,4H,5H,6H,7H-*pyrazolo[3,4-*b*]pyridin-6-on;
(+)-4-[4-(3,5-Dimethylphenoxy)-3-methoxyphenyl]-*2H,4H,5H,6H,7H*-pyrazolo[3,4-b]pyridin-6-on;
(-)-4-[4-(3,5-Dimethylphenoxy)-3-methoxyphenyl]-*2H,4H,5H,6H,7H*-pyrazolo[3,4-b]pyridin-6-on;
(+)-4-{3-Methoxy-4-[3-methyl-5-(trifluormethyl)phenoxy]phenyl}-*2H,4H,5H,6H,7H-*pyrazolo[3,4-*b*]pyridin-6-on;
(-)-4-{3-Methoxy-4-[3-methyl-5-(trifluormethyl)phenoxy]phenyl}*-2H,4H,5H,6H,7H-*pyrazolo[3,4-*b*]pyridin-6-on;
(+)-4-{4-[3-(Difluormethyl)-5-(trifluormethyl)-phenoxy]-3-methoxyphenyl}-*2H,4H,5H,6H,7H-*pyrazolo[3,4-*b*]pyridin-6-on; und
(-)-4-{4-[3-(Difluormethyl)-5-(trifluormethyl)-phenoxy]-3-methoxyphenyl}-*2H,4H,5H,6H,7H-*pyrazolo[3,4-*b*]pyridin-6-on.

8. Die Verbindung nach Anspruch 1, nämlich
(+)-4-{4-[3,5-Bis(trifluormethyl)phenoxy]-3-methoxyphenyl}-*2H,4H,5H,6H,7H-*pyrazolo[3,4-*b*]pyridin-6-on; oder
(-)-4-{4-[3,5-Bis(trifluormethyl)phenoxy]-3-methoxyphenyl}-*2H,4H,5H,6H,7H-*pyrazolo[3,4-*b*]pyridin-6-on.

9. Medikament, **dadurch gekennzeichnet, dass** es eine Verbindung der Formel I nach einem der Ansprüche 1 bis 8 oder ein pharmazeutisch verträgliches Salz davon umfasst.

10. Verbindung nach einem der Ansprüche 1 bis 8 oder ein pharmazeutisch verträgliches Salz davon zur Verwendung in der Therapie.

11. Verbindung nach einem der Ansprüche 1 bis 8 oder ein pharmazeutisch verträgliches Salz davon zur Verwendung bei der Behandlung von ERRα-vermitteltem Krebs.

12. Verbindung nach einem der Ansprüche 1 bis 8 oder ein pharmazeutisch verträgliches Salz davon zur Verwendung bei der Behandlung von mindestens einem Zustand, ausgewählt aus: Lungenkrebs, Melanom, Endometriumkrebs und akuter myeloischer Leukämie.

13. Verbindung nach einem der Ansprüche 1 bis 8 oder ein pharmazeutisch verträgliches Salz davon zur Verwendung bei der Behandlung von mindestens einem Zustand, ausgewählt aus: Brustkrebs; Blasenkrebs; Prostatakrebs; Bauchspeicheldrüsenkrebs; Darmkrebs; und Eierstockkrebs.

14. Verbindung nach einem der Ansprüche 1 bis 8 oder ein pharmazeutisch verträgliches Salz davon zur Verwendung bei der Behandlung von Diabetes mellitus Typ II.

15. Arzneimittel, das eine Verbindung der Formel I nach einem der Ansprüche 1 bis 8 oder ein pharmazeutisch verträgliches Salz davon und einen oder mehrere pharmazeutisch verträgliche Exzipienten umfasst, insbesondere wobei das Arzneimittel weiter mindestens einen zusätzlichen therapeutischen Wirkstoff umfasst.

## Revendications

1. Composé selon la Formule I 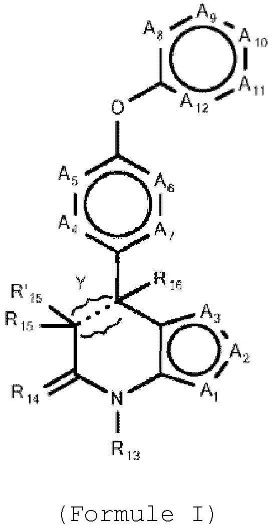 ou un sel pharmaceutiquement acceptable de celui-ci dans lequel
Y est une liaison carbone-carbone simple ou une double liaison carbone-carbone, à condition que lorsque Y est une double liaison carbone-carbone, et R₁₆ ne soient pas présents ;
l'une des trois positions A₁-A₃ est soit un S soit un NR_{A}, les deux positions restantes A₁-A₃ sont un N ou un CR₁, un CR₂, un CR₃, respectivement ;
R_{A} est un H ou un méthyle ;
R₁-R₃ sont indépendamment un H, un méthyle, un amino ou un halogène ;
A₄-A₇ sont un CR₄, un CR₅, un CR₆ et un CR₇, respectivement ;
A₈-A₁₂ sont un N ou un CR₈, un CR₉, un CR₁₀, un CR₁₁ et un CR₁₂, respectivement, à condition que pas plus de deux des cinq positions A₈-A₁₂ puissent être simultanément un N ; R₄-R₇ sont indépendamment un H, un halogène, un C(1-3)alcoxy, un C(1-3)alkyle ;
R₈-R₁₂ sont indépendamment un H, un halogène, un C(1-3)alcoxy, un C(1-4)alkyle, un cyano, un nitro, un - C(=O)OR₁₇, un hydroxyle, un C(3-6)cycloalkyle, un benzyle, un phényle, un -SF₅, un bicyclo[1.1.1]pentane ;
ou R₉ et soit R₈ soit R₁₀ sont fusionnés et forment un noyau aromatique ou non aromatique de cinq à sept chaînons contenant deux à sept atomes de carbone et zéro à trois hétéroatomes ; avec tous les atomes de carbone facultativement substitués par un ou plusieurs méthyle, halogène ou hydroxyle ;
R₁₃ est un H ou un méthyle ;
R₁₄ est un NH, un O ou un S ;
est un H, un halogène, un C(1-4)alkyle, un -C(=O)OR₁₇ ou un -C(=O)NR₁₇R₁₇ ;
R₁₅ est un H, un halogène ou un C(1-4)alkyle ;
R₁₆ est un H ; et,
R₁₇ est un H, un méthyle ou un éthyle.

2. Composé selon la revendication 1, dans lequel A₁ est un N, A₂ est un NR_{A} et A₃ est un CR₃, en particulier dans lequel A₁ est un N, A₂ est un NH et A₃ est un CH.

3. Composé selon la revendication 1 ou 2, dans lequel R₅ est un C(1-3)alcoxy et R₄, R₆ et R₇ sont un H, en particulier dans lequel R₅ est un méthoxy et R₄, R₆ et R₇ sont un H.

4. Composé selon l'une quelconque des revendications 1 à 3, dans lequel A₈-A₁₂ sont un CR₈, un CR₉, un CR₁₀, un CR₁₁ et un CR₁₂ respectivement, en particulier dans lequel R₈-R₁₂ sont indépendamment un H, un C(1-4)alkyle, un halogène, un hydroxyle ou un C(1-3)alcoxy, plus particulièrement dans lequel R₉ et R₁₁ sont un C(1-4)alkyle et R₈, R₁₀ et R₁₂ sont un H, plus particulièrement dans lequel R₉ et R₁₁ sont un CF₃ et R₈, R₁₀ et R₁₂ sont un H.

5. Composé selon l'une quelconque des revendications 1 à 4, dans lequel R₁₃ est un H ; et/ou dans lequel R₁₄ est un O ; et/ou dans lequel Y est une liaison carbone-carbone simple.

6. Composé selon l'une quelconque des revendications 1 à 5, dans lequel R₁₅ et R'₁₅ sont un H.

7. Composé selon la revendication 1, qui est choisi dans le groupe constitué de :
4-[3-méthoxy-4-(3-méthylphénoxy)phényl]-*2H,4H,5H,6H,7H-*pyrazolo[3,4-*b*]pyridin-6-one ;
4-(2-méthoxy-4-{6-oxo-*2H,4H,5H,6H,7H*-pyrazolo[3,4-*b*]pyridin-4-yl}phénoxy)-3-(trifluorométhyl)benzonitrile ;
4-[3-méthoxy-4-(2-nitrophénoxy)phényl]-*2H,4H,5H,6H,7H-*pyrazolo[3,4-*b*]pyridin-6-one ;
4-{3-méthoxy-4-[2-(trifluorométhyl)phénoxy]phényl}-*2H,4H,5H,6H,7H*-pyrazolo[3,4-*b*]pyridin-6-one ;
4-{3-méthoxy-4-[4-(trifluorométhyl)phénoxy]phényl}-*2H,4H,5H,6H,7H*-pyrazolo[3,4-*b*]pyridin-6-one ;
4-{3-méthoxy-4-[3-(trifluorométhyl)phénoxy]phényl}-*2H,4H,5H,6H,7H*-pyrazolo[3,4-*b*]pyridin-6-one ;
4-[4-(2-bromophénoxy)-3-méthoxyphényl]-*2H,4H,5H,6H,7H-*pyrazolo[3,4-*b*]pyridin-6-one ;
4-[3-méthoxy-4-(2-méthylphénoxy)phényl]-*2H,4H,5H,6H,7H-*pyrazolo[3,4-*b*]pyridin-6-one ;
2-(2-méthoxy-4-{6-oxo-*2H,4H,5H,6H,7H*-pyrazolo[3,4-*b*]pyridin-4-yl}phénoxy)benzonitrile ;
4-[4-(2-*tert*-butylphénoxy)-3-méthoxyphényl]-*2H,4H,5H,6H,7H*-pyrazolo[3,4-*b*]pyridin-6-one ;
4-[4-(2-chlorophénoxy)-3-méthoxyphényl]-2H,4H,5H,6H,7H-pyrazolo[3,4-*b*]pyridin-6-one ;
4-{3-méthoxy-4-[2-(trifluorométhoxy)phénoxy]phényl}-*2H,4H,5H,6H,7H*-pyrazolo[3,4-*b*]pyridin-6-one ;
4-[3-méthoxy-4-(2-méthoxyphénoxy)phényl]-*2H,4H,5H,6H,7H-*pyrazolo[3,4-*b*]pyridin-6-one ;
4-[4-(3-bromophénoxy)-3-méthoxyphényl]-*2H,4H,5H,6H,7H-*pyrazolo[3,4-*b*]pyridin-6-one ;
4-[4-(3-*tert*-butylphénoxy)-3-méthoxyphényl]-*2H,4H,5H,6H,7H*-pyrazolo[3,4-*b*]pyridin-6-one ;
4-[4-(3-chlorophénoxy)-3-méthoxyphényl]-*2H,4H,5H,6H,7H-*pyrazolo[3,4-*b*]pyridin-6-one ;
4-{3-méthoxy-4-[3-(trifluorométhoxy)phénoxy]phényl}-*2H,4H,5H,6H,7H*-pyrazolo[3,4-*b*]pyridin-6-one ;
4-[3-méthoxy-4-(3-méthoxyphénoxy)phényl]-*2H,4H,5H,6H,7H-*pyrazolo[3,4-*b*]pyridin-6-one ;
3-(2-méthoxy-4-{6-oxo-*2H,4H,5H,6H,7H-*pyrazolo[3,4-*b*]pyridin-4-yl}phénoxy)benzoate de méthyle ;
4-[4-(2-éthylphénoxy)-3-méthoxyphényl]-*2H,4H,5H,6H,7H-*pyrazolo[3,4-*b*]pyridin-6-one ;
4-[4-(3-éthylphénoxy)-3-méthoxyphényl]-*2H,4H,5H,6H,7H-*pyrazolo[3,4-*b*]pyridin-6-one ;
4-[3-méthoxy-4-(2-propylphénoxy)phényl]-*2H,4H,5H,6H,7H-*pyrazolo[3,4-*b*]pyridin-6-one ;
4-{3-méthoxy-4-[2-(propan-2-yl)phénoxy]phényl}-*2H,4H,5H,6H,7H*-pyrazolo[3,4-*b*]pyridin-6-one ;
4-[3-méthoxy-4-(2-phénylphénoxy)phényl]-*2H,4H,5H,6H,7H-*pyrazolo[3,4-*b*]pyridin-6-one ;
4-[4-(2-cyclopentylphénoxy)-3-méthoxyphényl]-*2H,4H,5H,6H,7H*-pyrazolo[3,4-*b*]pyridin-6-one ;
4-[4-(2-benzylphénoxy)-3-méthoxyphényl]-*2H,4H,5H,6H,7H-*pyrazolo[3,4-*b*]pyridin-6-one ;
4-[4-(2-*tert*-butyl-4-méthoxyphénoxy)-3-méthoxyphényl]-*2H,4H,5H,6H,7H*-pyrazolo[3,4-*b*]pyridin-6-one ;
4-[4-(2-cyclopropylphénoxy)-3-méthoxyphényl]-*2H,4H,5H,6H,7H*-pyrazolo[3,4-*b*]pyridin-6-one ;
4-[4-(3-*tert-*butyl-4-hydroxyphénoxy)-3-méthoxyphényl]-*2H,4H,5H,6H,7H*-pyrazolo[3,4-*b*]pyridin-6-one ;
4-{3-méthoxy-4-[3-(propan-2-yl)phénoxy]phényl}-*2H,4H,5H,6H,7H*-pyrazolo[3,4-*b*]pyridin-6-one ;
4-[4-(3-cyclopropylphénoxy)-3-méthoxyphényl]-*2H,4H,5H,6H,7H*-pyrazolo[3,4-*b*]pyridin-6-one ;
4-[4-(2-*tert*-butyl-4-éthylphénoxy)-3-méthoxyphényl]-*2H,4H,5H,6H,7H*-pyrazolo[3,4-*b*]pyridin-6-one ;
4-[4-(4-bromo-2-*tert*-butylphénoxy)-3-méthoxyphényl]-*2H,4H,5H,6H,7H*-pyrazolo[3,4-*b*]pyridin-6-one ;
4-[4-(2-*tert*-butyl-4-méthylphénoxy)-3-méthoxyphényl]-*2H,4H,5H,6H,7H*-pyrazolo[3,4-*b*]pyridin-6-one ;
3-*tert*-butyl-4-(2-méthoxy-4-{6-oxo-*2H,4H,5H,6H,7H-*pyrazolo[3,4-*b*]pyridin-4-yl}phénoxy)benzoate de méthyle ;
4-(2-méthoxy-4-{6-oxo-*2H,4H,5H,6H,7H*-pyrazolo[3,4-*b*]pyridin-4-yl}phénoxy)naphtalène-1-carboxylate de méthyle ;
4-[4-(2,4-di-*tert-*butylphénoxy)-3-méthoxyphényl]-*2H,4H,5H,6H,7H*-pyrazolo[3,4-*b*]pyridin-6-one ;
4-{3-méthoxy-4-[3-méthoxy-5-(trifluorométhyl)phénoxy]phényl}-*2H*,*4H*,*5H*,*6H*,*7H-*pyrazolo[3,4-*b*]pyridin-6-one ;
3-tert-butyl-4-(2-méthoxy-4-{6-oxo-*2H*,*4H*,*5H*,*6H*,*7H-*pyrazolo[3,4-*b*]pyridin-4-yl}phénoxy)benzonitrile ;
4-{3-méthoxy-4-[2-(pentafluoro-λ⁶-sulfanyl)phénoxy]phényl}-*2H*,*4H*,*5H*,*6H*,*7H*-pyrazolo[3,4-*b*]pyridin-6-one ;
3-(2-méthoxy-4-{6-oxo-*2H*,*4H*,*5H*,*6H*,*7H*-pyrazolo[3,4-*b*]pyridin-4-yl}phénoxy)-5-(trifluorométhyl)benzoate de méthyle ;
4-{4-[3-bromo-5-(trifluorométhyl)phénoxy]-3-méthoxyphényl}-*2H*,*4H*,*5H*,*6H*,*7H*-pyrazolo[3,4-*b*]pyridin-6-one ;
4-{4-[3,5-bis(trifluorométhyl)phénoxy]-3-méthoxyphényl}-*2H*,*4H*,*5H*,*6H*,*7H*-pyrazolo[3,4-*b*]pyridin-6-one ;
4-[4-(3,5-di-*tert*-butylphénoxy)-3-méthoxyphényl]-*2H*,*4H*,*5H*,*6H*,*7H*-pyrazolo[3,4-*b*]pyridin-6-one ;
4-[4-(2-{bicyclo[1.1.1]pentan-1-yl}phénoxy)-3-méthoxyphényl]-*2H*,*4H*,*5H*,*6H*,*7H*-pyrazolo[3,4-*b*]pyridin-6-one ;
4-(2-méthoxy-4-{6-oxo-*2H*,*4H*,*5H*,*6H*,*7H*-pyrazolo[3,4-*b*]pyridin-4-yl}phénoxy)naphtalène-1-carbonitrile ;
4-[4-(2,3-dihydro-*1H*-indén-4-yloxy)-3-méthoxyphényl]-*2H*,*4H*,*5H*,*6H*,*7H*-pyrazolo[3,4-*b*]pyridin-6-one ;
4-{4-[(2,2-diméthyl-2,3-dihydro-1-benzofuran-7-yl)oxy]-3-méthoxyphényl}-*2H*,*4H*,*5H*,*6H*,*7H*-pyrazolo[3,4-*b*]pyridin-6-one ;
4-[4-(2,3-dihydro-1*H*-indén-5-yloxy)-3-méthoxyphényl]-*2H*,*4H*,*5H*,*6H*,*7H*-pyrazolo[3,4-*b*]pyridin-6-one ;
8-(2-méthoxy-4-{6-oxo-*2H,4H,5H,6H,7H*-pyrazolo[3,4-*b*]pyridin-4-yl}phénoxy)quinoline-5-carboxylate de méthyle ;
4-(3-méthoxy-4-phénoxyphényl)-*2H,4H,5H,6H,7H-*pyrazolo[3,4-*b*]pyridin-6-one ;
4-{3-bromo-4-[3-méthoxy-5-(trifluorométhyl)phénoxy]phényl}-*2H,4H,5H,6H,7H-*pyrazolo[3,4-*b*]pyridin-6-one ;
3-*tert*-butyl-4-(4-{6-oxo-*2H,4H,5H,6H,7H*-pyrazolo[3,4-*b*]pyridin-4-yl}phénoxy)benzonitrile ;
4-{4-[3-méthoxy-5-(trifluorométhyl)phénoxy]phényl}-*2H,4H,5H,6H,7H*-pyrazolo[3,4-*b*]pyridin-6-one ;
3-*tert*-butyl-4-(2-méthyl-4-{6-oxo-*2H,4H,5H,6H,7H-*pyrazolo[3,4-*b*]pyridin-4-yl}phénoxy)benzonitrile ;
4-{4-[3-méthoxy-5-(trifluorométhyl)phénoxy]-3-méthylphényl}-*2H,4H,5H,6H,7H*-pyrazolo[3,4-*b*]pyridin-6-one ;
4-{3-fluoro-4-[3-méthoxy-5-(trifluorométhyl)phénoxy]phényl}-*2H,4H,5H,6H,7H-*pyrazolo[3,4-*b*]pyridin-6-one ;
4-{4-[2-(pentafluoro-λ⁶-sulfanyl)phénoxy]-3-(trifluorométhoxy)phényl}-*2H*,*4H*,*5H*,*6H*,*7H*-pyrazolo[3,4-*b*]pyridin-6-one ;
4-{4-[2-(pentafluoro-λ⁶-sulfanyl)phénoxy]phényl}-*2H*,*4H*,*5H*,*6H*,*7H*-pyrazolo[3,4-*b*]pyridin-6-one ;
4-[3-(trifluorométhoxy)-4-[2-(trifluorométhyl)phénoxy]phényl]-*2H*,*4H*,*5H*,*6H*,*7H-*pyrazolo[3,4-*b*]pyridin-6-one ;
acide 3-*tert*-butyl-4-(2-méthoxy-4-{6-oxo-*2H*,*4H*,*5H*,*6H*,*7H-*pyrazolo[3,4-*b*]pyridin-4-yl}phénoxy)benzoïque ;
acide 3-(2-méthoxy-4-{6-oxo-*2H*,*4H*,*5H*,*6H*,*7H*-pyrazolo[3,4-*b*]pyridin-4-yl}phénoxy)-5-(trifluorométhyl)benzoïque ;
4-{3-méthoxy-4-[3-méthyl-5-(trifluorométhyl)phénoxy]phényl}-*2H,4H,5H,6H,7H-*pyrazolo[3,4-*b*]pyridin-6-one ;
4-[4-(3,5-diméthylphénoxy)-3-méthoxyphényl]-*2H,4H,5H,6H,7H*-pyrazolo[3,4-*b*]pyridin-6-one ;
4-{3-méthoxy-4-[2-méthyl-4-(trifluorométhyl)phénoxy]phényl}-*2H,4H,5H,6H,7H-*pyrazolo[3,4-*b*]pyridin-6-one ;
4-[4-(2,4-diméthylphénoxy)-3-méthoxyphényl]-*2H,4H,5H,6H,7H*-pyrazolo[3,4-*b*]pyridin-6-one ;
4-[4-(2-*tert*-butyl-4-éthylphénoxy)-3-méthoxyphényl]-*2H,4H,5H,6H,7H*-pyrazolo[3,4-*b*]pyridin-6-one ;
4-[4-(2-*tert*-butyl-4-méthoxyphénoxy)-3-méthoxyphényl]-*2H,4H,5H,6H,7H*-pyrazolo[3,4-*b*]pyridin-6-one ;
4-[4-(2-*tert-*butyl-4-méthylphénoxy)-3-méthoxyphényl]-*2H,6H,7H*-pyrazolo[3,4-*b*]pyridin-6-one ;
4-[4-(4-bromo-2-*tert*-butylphénoxy)-3-méthoxyphényl]-*2H,6H,7H*-pyrazolo[3,4-*b*]pyridin-6-one ;
4-(2-méthoxy-4-{6-oxo-*2H,4H,5H,6H,7H*-pyrazolo[3,4-*b*]pyridin-4-yl}phénoxy)-3-(trifluorométhyl)benzonitrile ;
4-{4-[3,5-bis(trifluorométhyl)phénoxy]-3-méthoxyphényl}-6-oxo-*2H*,*4H*,*5H*,*6H*,*7H*-pyrazolo[3,4-*b*]pyridine-5-carboxylate de méthyle ;
4-{4-[3,5-bis(trifluorométhyl)phénoxy]-3-méthoxyphényl}-6-oxo-*2H*,*4H*,*5H*,*6H*,*7H*-pyrazolo[3,4-*b*]pyridine-5-carboxylate d'éthyle ;
4-{4-[3,5-bis(trifluorométhyl)phénoxy]-3-méthoxyphényl}-*N*,*N*-diméthyl-6-oxo-*2H*,*4H*,*5H*,*6H*,*7H*-pyrazolo[3,4-*b*]pyridine-5-carboxamide ;
4-{4-[3,5-bis(trifluorométhyl)phénoxy]-3-méthoxyphényl}-*N,N*-diéthyl-6-oxo-*2H*,*4H*,*5H*,*6H*,*7H*-pyrazolo[3,4-*b*]pyridine-5-carboxamide ;
4-{4-[3,5-bis(trifluorométhyl)phénoxy]-3-méthoxyphényl}-*1H,2H,3H,4H,5H-*pyrrolo[3,4-*b*]pyridin-2-one ;
4-{4-[3-(difluorométhyl)-5-(trifluorométhyl)phénoxy]-3-méthoxyphényl}-*2H*,*4H*,*5H*,*6H*,*7H*-pyrazolo[3,4-*b*]pyridin-6-one ;
4-{4-[2-(difluorométhyl)-4-(trifluorométhyl)phénoxy]-3-méthoxyphényl}-*2H*,*4H*,*5H*,*6H*,*7H*-pyrazolo[3,4-*b*]pyridin-6-one ;
(+)-4-{4-[3,5-bis(trifluorométhyl)phénoxy]-3-méthoxyphényl}-*2H*,*4H*,*5H*,*6H*,*7H*-pyrazolo[3,4-*b*]pyridin-6-one ;
(-)-4-{4-[3,5-bis(trifluorométhyl)phénoxy]-3-méthoxyphényl}-*2H*,*4H*,*5H*,*6H*,*7H*-pyrazolo[3,4-*b*]pyridin-6-one ;
(+)-4-[4-(3,5-diméthylphénoxy)-3-méthoxyphényl]-*2H,4H,5H,6H,7H*-pyrazolo[3,4-*b*]pyridin-6-one ;
(-)-4-[4-(3,5-diméthylphénoxy)-3-méthoxyphényl]-*2H,4H,5H,6H,7H*-pyrazolo[3,4-b]pyridin-6-one ;
(+)-4-{3-méthoxy-4-[3-méthyl-5-(trifluorométhyl)phénoxy]phényl}-*2H,4H,5H,6H,7H-*pyrazolo[3,4-*b*]pyridin-6-one ;
(-)-4-{3-méthoxy-4-[3-méthyl-5-(trifluorométhyl)phénoxy]phényl}-*2H,4H,5H,6H,7H-*pyrazolo[3,4-*b*]pyridin-6-one ;
(+)-4-{4-[3-(difluorométhyl)-5-(trifluorométhyl)phénoxy]-3-méthoxyphényl}-*2H,4H,5H,6H,7H*-pyrazolo[3,4-*b*]pyridin-6-one ; et,
(-)-4-{4-[3-(difluorométhyl)-5-(trifluorométhyl)phénoxy]-3-méthoxyphényl}-*2H,4H,5H,6H,7H*-pyrazolo[3,4-*b*]pyridin-6-one.

8. Composé selon la revendication 1, qui est de la (+)-4-{4-[3,5-bis(trifluorométhyl)phénoxy]-3-méthoxyphényl}-*2H*,*4H*,*5H*,*6H*,*7H*-pyrazolo[3,4-*b*]pyridin-6-one ; ou de la (-)-4-{4-[3,5-bis(trifluorométhyl)phénoxy]-3-méthoxyphényl}-*2H*,*4H*,*5H*,*6H*,*7H*-pyrazolo[3,4-*b*]pyridin-6-one.

9. Médicament **caractérisé en ce qu'**il comprend un composé de Formule I selon l'une quelconque des revendications 1 à 8 ou un sel pharmaceutiquement acceptable de celui-ci.

10. Composé selon l'une quelconque des revendications 1 à 8 ou un sel pharmaceutiquement acceptable de celui-ci pour une utilisation en thérapie.

11. Composé selon l'une quelconque des revendications 1 à 8 ou un sel pharmaceutiquement acceptable de celui-ci pour une utilisation dans le traitement d'un cancer médié par ERRα.

12. Composé selon l'une quelconque des revendications 1 à 8 ou un sel pharmaceutiquement acceptable de celui-ci pour une utilisation dans le traitement d'au moins une affection choisie parmi : cancer du poumon ; mélanome ; cancer de l'endomètre ; et leucémie myéloïde aiguë.

13. Composé selon l'une quelconque des revendications 1 à 8 ou un sel pharmaceutiquement acceptable de celui-ci pour une utilisation dans le traitement d'au moins une affection choisie parmi : cancer du sein ; cancer de la vessie ; cancer de la prostate ; cancer du pancréas ; cancer colorectal ; et cancer de l'ovaire.

14. Composé selon l'une quelconque des revendications 1 à 8 ou un sel pharmaceutiquement acceptable de celui-ci pour une utilisation dans le traitement du diabète sucré de type II.

15. Composition pharmaceutique qui comprend un composé de Formule I selon l'une quelconque des revendications 1 à 8 ou un sel pharmaceutiquement acceptable de celui-ci et un ou plusieurs excipients pharmaceutiquement acceptables, en particulier laquelle composition pharmaceutique comprend en outre au moins un agent thérapeutiquement actif additionnel.
